# EUROPEAN PATENT APPLICATION

(11) **EP 4 047 079 A1**
(43) Date of publication of application: **24.08.2022**
(21) Application number: 20877478.6
(22) Date of filing: 15.10.2020
(51) Int. Cl.: C12M 3/00, C12N 5/071, A61L 27/18, A61L 27/38, A61L 27/56

(54) **CELL SHEET PRODUCTION DEVICE AND CELL SHEET**

(30) Priority: 16.10.2019 JP 2019189611; 24.01.2020 JP 2020010229
(71) Applicant: RIKEN, Wako-shi Saitama 351-0198 (JP); Mizuta Seisakusho,inc., Akashi-shi, Hyogo 673-0023 (JP)
(72) Inventor: HORI, Takeshi, Wako-shi, Saitama 351-0198 (JP); IWATA, Hiroo, Wako-shi, Saitama 351-0198 (JP); KUROSAWA, Osamu, Wako-shi, Saitama 351-0198 (JP); TANI, Nobutaka, Wako-shi, Saitama 351-0198 (JP); MIZUTA, Taro, Akashi-shi, Hyogo 673-0023 (JP); ISHIHARA, Kouhei, Akashi-shi, Hyogo 673-0023 (JP)
(74) Representative: Graf von Stosch Patentanwaltsgesellschaft mbH
(86) International application number: PCT/JP2020/038938
(87) International publication number: WO 2021/075502

(57) **Abstract**

An aspect of the present invention makes it possible to easily observe both surfaces of a cell sheet in a condition in which the cell sheet is at a stable position in a culture medium. A cell sheet production device (100) includes: a container (20); and a support unit (10) removably held in the container (20), the support unit (10) including a mesh sheet (2) and a base (3) which holds the mesh sheet (2) such that the mesh sheet (2) floats from a bottom surface of the container (20), the support unit (10) being held in the container (20) such that the support unit is at a fixed position in vertical and horizontal directions in a culture medium.

## Description

### Technical Field

The present invention relates to a cell sheet production device, and a cell sheet.

### Background Art

Cell sheets are practically used as skin sheets in treating burned areas, and the like sheets. In the future, cell sheets may also be used as cardiomyocyte sheets which are to be grafted on heart failure patients or as islet cell sheets which are to be grafted on diabetic patients. Cell sheets are thus expected to be applied to many patients.

Meanwhile, attempts have been made to reconstruct a tissue structure similar to an in-vivo tissue structure by means of a cell sheet which is made of a plurality of cell layers. Therefore, the importance of a method for preparing a cell sheet in which cells are three-dimensionally arranged has been recognized. In a case where a cell sheet is prepared by a method for preparing a cell sheet with use of an existing temperature-responsive culture dish, the cell sheet thus obtained is fragile and accordingly often difficult to handle (see Non-Patent Literatures 1 and 2). In a case where a cell sheet which is made of a plurality of cell layers (dermal and epidermal layers) is to be prepared on a dish, a mature epidermal layer forms a kind of barrier that is called a tight junction. Therefore, it is concerned that nutrient supply to the inside of the cell sheet may be impaired.

Further, a method for preparing a cell sheet on a non-cellular sheet-shaped support has been proposed. However, in a case where the support is not compatible with cells in an affected area, it is not possible to attach an exposed surface of the support to the affected area in such a method. That is, an attachment surface of the cell sheet to the affected area is limited to one surface. In a case where a keratinocyte layer (epidermal layer) is provided on a fibroblast layer (dermal layer) in a cell sheet which is to be attached to an affected area, it is extremely inconvenient that the attachment surface is limited as described above. This is because in a case where a cell sheet is grafted, it is necessary to cause a dermal layer-side surface of the cell sheet to adhere to an affected area.

There is also another known method according to which a cell sheet made of a plurality of cell layers is prepared on a porous membrane (e.g., a polycarbonate porous membrane) (see Non-Patent Literature 3). In a cell sheet which is prepared by this method, nutrient supply to cells via pores of the porous membrane is expected. Therefore, nutrient supply to cells is better than that in a case where a multilayered cell sheet is prepared on a dish. However, even in a case where a cell sheet is prepared on a porous membrane, the cell sheet thus prepared has a single attachment surface for attachment to an affected area. Accordingly, use of such a cell sheet for grafting is difficult. Therefore, the cell sheet which has been prepared by the above method is mainly used in permeability tests of compounds, and the like.

According to another method which has been proposed, a cell sheet is prepared with use of a nanofiber sheet which is made of, for example, gelatin, polylactic acid (PLA), or a poly(lactic-co-glycolic acid) copolymer (PLGA) (see Non-Patent Literature 4). In this method, a sheet made of nanofibers is prepared by an electrospinning method, and cells are cultured on the sheet. Such a method has a problem in that it is difficult to accurately control respective positions of the nanofibers by electrospinning. Because of this irregularity problem of the nanofibers, it may be difficult to obtain a highly reproducible result for a cell sheet which has been prepared.

It has also been proposed to use a micromesh sheet for preparation of a cell sheet. As a method using a micromesh sheet, for example, the following methods have been reported: (i) a method according to which a two-dimensional cell sheet is prepared by culturing cells on a micromesh sheet; (ii) a method according to which spherical trophoblast-like cells are prepared by culturing iPS cells on a micromesh sheet; and the like method (see Patent Literatures 1 and 2 and Non-Patent Literature 5). According to a technique which has been developed as an application of such a cell culture method with use of a micromesh sheet, a three-dimensional cell sheet is prepared by culturing HepG2 cells of a hepatic cell line on a micromesh sheet. It is suggested that in this three-dimensional cell sheet, some of cell functions are improved as compared to those of three-dimensional spheroids, and also that the three-dimensional cell sheet is excellent in cell observation and nutrient supply and in compatibility with fluidic devices (see Non-Patent Literature 6).

### Citation List

### [Patent Literature]

[Patent Literature 1]
   Pamphlet of International Publication No. WO 2015/005349 (Publication Date: January 15, 2015)
[Patent Literature 2]
   Japanese Patent Application Publication Tokukai No. 2019-50773 (Publication Date: April 4, 2019)

### [Non-patent Literature]

[Non-patent Literature 1]
   Green H, Kehinde O, Thomas J. Growth of cultured human epidermal cells into multiple epithelia suitable for grafting. Proc Natl Acad Sci USA. 1979; 76(11): 5665-8.
[Non-patent Literature 2]
   Yamada N, Okano T, Sakai H, Karikusa F, Sawasaki Y, Sakurai Y. Thermo-responsive polymeric surfaces; control of attachment and detachment of cultured cells. Die Makromolekulare Chemie, Rapid Communications. 1990; 11(11): 571-6.
[Non-patent Literature 3]
   Kojima H, Ishii I, Nakata S, Konishi H. Dose-response Evaluation Using an Epidermal Model, an Alternative to Skin Irritation Testing. Alternatives to Animal Testing and Experimentation. 2006; 11(3): 177-84.
[Non-patent Literature 4]
   Li J, Minami I, Shiozaki M, Yu L, Yajima S, Miyagawa S, et al. Human Pluripotent Stem Cell-Derived Cardiac Tissue-like Constructs for Repairing the Infarcted Myocardium. Stem Cell Reports. 2017; 9(5): 1546-59.
[Non-patent Literature 5]
   Okeyo KO, Kurosawa O, Yamazaki S, Oana H, Kotera H, Nakauchi H, et al. Cell Adhesion Minimization by a Novel Mesh Culture Method Mechanically Directs Trophoblast Differentiation and Self-Assembly Organization of Human Pluripotent Stem Cells. Tissue Eng Part C Methods. 2015; 21(10): 1105-15.
[Non-patent Literature 6]
   Hori T, Kurosawa O. A Three-dimensional Cell Culture Method with a Micromesh Sheet and Its Application to Hepatic Cells. Tissue Eng Part C Methods. 2018.

### Summary of Invention

### Technical Problem

In a method for producing a cell sheet with use of a micromesh sheet, first, a mesh sheet is set in a container such that the mesh sheet is floated in a culture medium for culturing cells. Next, the cells are seeded on the micromesh. Then, the cells which have been seeded on the micromesh sheet are cultured in the culture medium. In such a method for producing a cell sheet, both surfaces of the cell sheet need to be checked, with use of a microscope, for confirmation of a growth situation of cells which constitute the cell sheet.

For example, in a technique disclosed in Patent Literature 1, the micromesh sheet on which cells are to be seeded is set on a hanging member that has been installed in a container. In this configuration, it is difficult to (i) invert the cell sheet and culture the cells and (ii) invert the cell sheet and observe the cell sheet with use of a microscope (i.e., observe both surfaces).

Meanwhile, in a device disclosed in Patent Literature 2, the micromesh sheet is held by a groove which is provided on an inner wall of a container which contains a culture medium. In this configuration, it is difficult to separate the macro-mesh sheet from the container and place the micromesh sheet in a reversed state. Therefore, it is difficult to check both surfaces of the cell sheet with use of a microscope.

Difficulty involved in the above cell observation may be a problem particularly in a case where, for example, a cell sheet made of a plurality of cell layers is prepared.

A first object of an aspect of the present invention is to realize a cell sheet production device that, in order to check a growth situation of cells constituting a cell sheet, allows both surfaces of the cell sheet to be easily observed in a condition in which the cell sheet is at a stable position in a culture medium.

Further, a second object of an aspect of the present invention is to realize a cell sheet which can ensure a sufficient thickness and which can have improved strength.

### Solution to Problem

In order to solve the above problems, a cell sheet production device in accordance with an aspect of the present invention includes: a container configured to contain a culture medium for culturing cells; and a support unit configured to be removably held in the container, the support unit including: a mesh sheet that is a substrate to which the cells are caused to adhere and on which the cells are cultured; and a holding member which holds the mesh sheet such that the mesh sheet floats from a bottom surface of the container, the support unit being held in the container such that the support unit is at a fixed position in vertical and horizontal directions in the culture medium.

A cell sheet in accordance with an aspect of the present invention includes: at least two cell layers; and a mesh sheet between the two cell layers, the mesh sheet being a substrate to which cells are caused to adhere and on which the cells are cultured.

### Advantageous Effects of Invention

An aspect of the present invention makes it possible to realize a cell sheet production device that, in order to check a growth situation of cells in a cell sheet, allows both surfaces of the cell sheet to be easily observed in a condition in which the cell sheet is at a stable position in a culture medium. Further, an aspect of the present invention can realize a cell sheet which can ensure a sufficient thickness and which can have improved strength.

### Brief Description of Drawings

Fig. 1 is a view schematically illustrating a configuration of a cell sheet production device according to Configuration Example 1.
Fig. 2 is a view illustrating effects of the cell sheet production device illustrated in Fig. 1.
Fig. 3 is a view schematically illustrating a configuration of a cell sheet production device according to Variation 1.
Fig. 4 is a view schematically illustrating a configuration of a cell sheet production device according to Variation 2.
Fig. 5 is a view schematically illustrating a cross-section structure of a skin sheet which is prepared by a cell sheet production device in accordance with an embodiment of the present invention.
Fig. 6 is a view schematically illustrating a configuration of a support unit which is provided in a cell sheet production device according to Configuration Example 2.
Fig. 7 is a view schematically illustrating a configuration of a cell sheet production device according to Configuration Example 2.
Fig. 8 is a view schematically illustrating a configuration of a cell sheet production device according to Configuration Example 3.
Fig. 9 is a view schematically illustrating a configuration of a cell sheet production device according to Configuration Example 4.
Fig. 10 is a view schematically showing results of Example 1.
Fig. 11 is a view schematically showing results of Example 1.
Fig. 12 is a view schematically showing results of Example 2.
Fig. 13 is a view schematically showing results of Example 3.
Fig. 14 is a view schematically showing results of Example 4.
Fig. 15 is a view schematically showing results of Example 5.
Fig. 16 is a view schematically showing results of Example 5.
Fig. 17 is a view schematically showing results of Example 5.
Fig. 18 is a view schematically showing results of Example 6.
Fig. 19 is a view schematically showing results of Example 7.
Fig. 20 is a view schematically illustrating a configuration of a cell sheet production device according to Variation 3.
Fig. 21 is a view illustrating effects of the cell sheet production device according to Variation 3.

### Description of Embodiments

The following description will discuss embodiments of the present invention. Note, however, that the present invention is not limited to such embodiments. The present invention is not limited to arrangements described below, but may be altered in various ways by a skilled person within the scope of the claims. Any embodiment and example derived from a combination of technical means disclosed in differing embodiments and/or examples are also encompassed in the technical scope of the present invention. In addition, the content of all literatures listed herein is incorporated herein as reference literatures. Note that a numerical range "A to B" herein means "not less than A and not more than B".

### 1. Cell sheet production device

A cell sheet production device in accordance with the present embodiment assumes that a cell sheet is produced by placing a mesh sheet in a culture medium, and then growing, in the culture medium, cells which have been seeded on the mesh sheet. The cells can be grown along the shape of the mesh sheet, by placing the mesh sheet in the culture medium (culture solution).

The cell sheet production device in accordance with the present embodiment is configured to include a container in which a culture medium for culturing cells is contained, and a support unit. The support unit is removably held in the container, and has a mesh sheet and a holding member. The mesh sheet is a substrate to which the cells are caused to adhere and on which the cells are cultured. The holding member holds the mesh sheet such that the mesh sheet floats from a bottom surface of the container. The support unit is held in the container such that the support unit is at a fixed position in vertical and horizontal directions in the culture medium.

The phrase "hold the mesh sheet such that the mesh sheet floats from a bottom surface of the container" means holding the mesh sheet such that both surfaces of the mesh sheet are not in contact with the bottom surface of the container (preferably not in contact with an inner wall and the bottom surface of the container) and the both surfaces of the mesh sheet are in sufficient contact with the culture medium. The holding member is not limited to a specific configuration and may have any configuration, provided that the holding member holds the mesh sheet such that the mesh sheet floats from the bottom surface of the container.

The cell sheet production device in accordance with the present embodiment, which uses the above-described mesh sheet, improves convenience for cell observation by a user. More specifically, in the above configuration, the support unit is removably held in the container. Accordingly, the user can easily reverse the support unit, by taking out the support unit from the container, reversing the support member, and then putting the support member back in the container. When the support unit is reversed, the cell sheet is also reversed, the cell sheet having been formed on the mesh sheet which is held by the holding member of the support unit. Therefore, the above configuration makes it possible to easily observe, with use of a microscope, both surfaces of the cell sheet which has been formed on the mesh sheet.

Further, the support unit is held in the container such that the support unit is at a fixed position in the vertical and horizontal directions in the culture medium. Accordingly, during observation of the cell sheet with use of the microscope, no change occurs in the position of the support unit in the container, in other words, the position of the cell sheet which has been formed on the mesh sheet. Therefore, the above configuration allows for precise observation of the cell sheet.

Further, in the cell sheet production device in accordance with the present embodiment, preferably, the holding member detachably holds the mesh sheet. This makes it possible to easily separate, from the support unit, the cell sheet which has been formed on the mesh sheet. Therefore, it is easy to handle the cell sheet, for example, in grafting the cell sheet.

Further, the support unit is not limited to a specific configuration and may have any configuration, provided that the support unit can be held in the container such that the support unit is at a fixed position in the vertical and horizontal directions in the culture medium. Examples of the configuration of the above support unit include a configuration in which the holding member has a larger specific gravity than the above-described culture medium. Because the holding member has a higher specific gravity than the culture medium, the holding member does not float but sinks in the culture medium, and in particular, the position of the holding member in the vertical direction is stabilized. The specific gravity of the holding member can be set as appropriate in accordance with a type of the culture medium. For example, when the specific gravity of the culture medium is 1, the specific gravity of the holding member is preferably not less than 1.1, more preferably not less than 1.5, more preferably not less than 2.0, more preferably not less than 5.0, and most preferably not less than 10.0. Further, examples of a material constituting the holding member include polystyrene, polyester, polyacetal, polycarbonate, and polyvinyl chloride.

Further, the mesh sheet is not particularly limited, provided that the mesh sheet is structured such that cells can be seeded and grown. The mesh sheet is preferably a planar structure in which openings of a predetermined shape are regularly or irregularly arranged repeatedly. More preferably, the mesh sheet has a large number of polygonal openings in plan view. The shape of the openings of the mesh sheet is typically a polygonal shape, such as a triangular shape, quadrangular shape, or hexagonal shape, but the shape may be a circular shape, elliptical shape, or other polygonal shape.

Herein, a portion of the mesh sheet other than the openings of the mesh sheet is referred to as a frame or a frame portion. When the openings are micrometer-sized, the mesh sheet may be also referred to as a micromesh or the like. Note that the shape of the openings of the mesh sheet can be also said to be the shape of areas which are surrounded by the frame constituting the mesh sheet.

Each of the openings of the mesh sheet may be sized so that at least one cell to be cultured can pass through the opening. The description "the opening is sized so that at least one cell to be cultured can pass through the opening" means that the size of the openings and the size of the cells has a relation with which a cell can pass through an opening while the cell is deformed or not deformed. The opening may be sized so that a cell can pass through an opening while the cell is brought into contact with the opening or while the cell is not brought into contact with the mesh (that is, while the cell is deformed).

Preferably, the shape of the openings of the mesh sheet is a shape which is elongated in one direction. The "shape which is elongated in one direction" means a shape in which one axis (long axis) that is longer than another axis or other axes (short axis or axes) exists among a plurality of axis lines that define the shape. Examples of such a shape include a rectangle, a rhombus, and an ellipse. In a case where the shape of the openings is a rectangle, the opening has a long side corresponding to the long axis and a short side corresponding to the short axis. Alternatively, in a case where the shape of the openings is a rhombus, the longer diagonal of the two diagonals corresponds to the long axis and the shorter diagonal corresponds to the short axis. Examples of the "shape which is elongated in one direction" include: a rectangle whose long axis (long side) is much longer than the short side (short axis), and a rhombus or ellipse whose long axis is much longer than the short axis. In a case where the shape is a rectangle, the shape which is elongated in one direction has a ratio of the short side to the long side of 1:2 to 1:5, and preferably 1:2 to 1:10. In a case where the shape is a rhombus, the shape which is elongated in one direction has a ratio of the short axis to the long axis of 1:2 to 1:5, and preferably 1:2 to 1:10. In a case where the shape is an ellipse, the shape which is elongated in one direction has a ratio of the short axis to the long axis of 1:2 to 1:5, and preferably 1:2 to 1:10. The present invention certainly is not limited to such configurations. Note that the "shape which is elongated in one direction" is not limited to a rectangle, a rhombus, or an ellipse, and any shape may be used, provided that one of a plurality of axes that define the shape is much longer than the other axis or axes.

In a case where as in the above configurations, the openings of the mesh sheet each have the shape which is elongated in one direction, the openings each have a large wall surface which extends in one direction. There are many cells that adhere to the wall surface and grow, and the cells each grow in a similar orientation. Therefore, according to the above configuration, it is possible to control the orientation of growing cells so that the orientation is in the direction of elongation of the openings.

Further, the mesh sheet may be made of any material, provided that cells can adhere to the material and grow. It is possible to use, as the material of the mesh sheet, for example, a photocurable resin, a biocompatible material, or a biodegradable material.

When a photocurable resin is used, the mesh sheet can be prepared by a photolithography method. Examples of the photocurable resin include an acrylate compound, a methacrylate compound, an epoxy compound, an isocyanate compound, a thiol compound, and a silicone-based compound. Two or more of these photocurable resins may be used in combination. Specific examples of the photocurable resin include, but are not limited to, urethane acrylate, polyester acrylate, epoxy acrylate, poly(methyl(meth)acrylate), ethoxylated bisphenol A acrylate, aliphatic urethane acrylate, polyester acrylate, polyethylene terephthalate, polystyrene, polycarbonate, acrylic modified alicyclic epoxide, bifunctional alcohol ether type epoxide, acrylic silicone, acrylic dimethylsiloxane, and polydimethylsiloxane (PDMS).

When the mesh sheet is prepared by a photolithography method, it is possible to prepare a micromesh sheet with use of a positive resist which causes an exposed portion to be removed. Examples of the positive resist include: a diazonaphthoquinone (DNQ) novolac-based resin positive resist; a deprotection reaction type positive resist such as t-butoxycarbonyl, tetrahydropyran, phenoxyethyl, trimethylsilyl, and t-butoxycarbonylmethyl; and a depolymerization reaction type positive resist such as polyphthalaldehyde, polycarbonate, and polysilyerutel. In addition, tetramethylammonium hydroxide (TMAH), dimethyl sulfoxide (DMSO), melethyl ketone (MEK), γ-butyrolactone (GBL), ethyl lactate (EL), or the like are used as a developer.

Further, in a case where the material of the mesh sheet is a biocompatible material or a biodegradable material, a resultant cell sheet as is can be grafted on a living body together with the mesh sheet. Therefore, such a cell sheet is suitably used for regenerative medicine or drug development.

Examples of the biocompatible material include, but are not limited to, silicone, polyether block amide (PEBAX), polyurethane, silicone-polyurethane copolymers, ceramics, collagen, hydroxyapatite, nylon, polyethylene terephthalate, ultra-high molecular weight polyethylene such as GORE-TEX (Registered Trademark), polyvinyl chloride, and tissue-derived biomaterials. In addition, the mesh sheet may be made of a material other than a biocompatible material, and the mesh sheet may have a surface which has been treated with a biocompatible material.

Examples of the biodegradable material include, but are not limited to: polylactide (PLA), polyglycolide (PGA), polycaprolactone (PCL), and copolymers thereof; PHB-PHV-based (poly)alkanoic acids; polyesters; and natural polymers such as starch, cellulose, and chitosan and derivatives thereof.

The material of the mesh sheet is preferably polyester, and particularly preferably polyethylene terephthalate, among the above materials. Since polyethylene terephthalate has low toxicity, a resultant cell sheet can be grafted in vivo. Further, in order to suppress autofluorescence during fluorescence microscope observation, it is preferable that the polyethylene terephthalate be dyed black. Specific examples of the mesh sheet made of polyester include clothing fabrics AG001N0, AG00Z1N0, and AG00Z3N0 which are manufactured by Amaike Textile Industry Co., Ltd. Further, specific examples of the mesh sheet made of polyester that is dyed black include clothing fabrics AG001N1, AG00Z1N9, and AG00Z3N9 which are manufactured by Amaike Textile Industry Co., Ltd.

The mesh sheet may be preliminarily coated with a cell adhesion promoting material. The cell adhesion promoting material is used to fix cells to a culture support and to facilitate extension and growth of the cells. Examples of the cell adhesion promoting material include extracellular matrix proteins such as collagen, fibronectin, and laminin, and positively charged substances such as poly-L-lysine. Specifically, the cell adhesion promoting material can be Matrigel (registered trademark).

In addition, in the cell sheet production device in accordance with the present embodiment, the container is not particularly limited in configuration (e.g., shape), provided that the container can contain a culture medium for culturing cells. Examples of the container include dishes for cell culture, plates each having a plurality of wells (6-well plate, 12-well plate, etc.), and columns for cell culture.

### 2. Configuration Example 1 of cell sheet production device in accordance with present embodiment

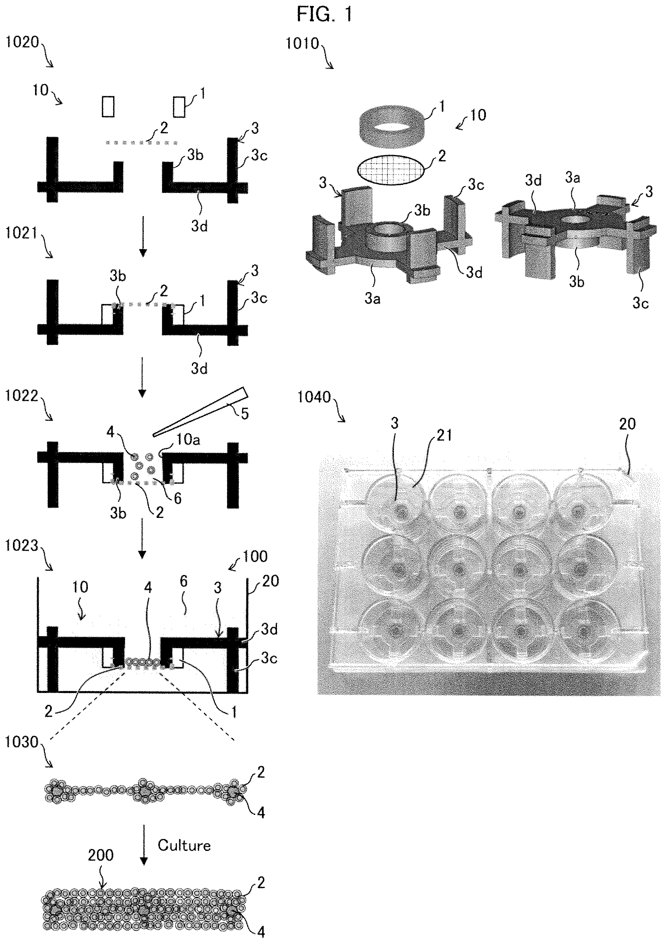

The following will discuss Configuration Example 1 of the cell sheet production device in accordance with the present embodiment. Fig. 1 is a view schematically illustrating a configuration of a cell sheet production device 100 according to Configuration Example 1. Fig. 1 includes 1010 which is a perspective view illustrating a configuration of a support unit 10 that is provided in the cell sheet production device 100. Fig. 1 further includes 1020 to 1023 which are views illustrating a procedure for a method for producing a cell sheet by using the cell sheet production device 100. Fig. 1 further includes 1030 which is a view illustrating a configuration of the cell sheet which has been prepared by using the cell sheet production device 100. Fig. 1 further includes 1040 which shows an image of an example of a specific configuration of the cell sheet production device 100.

As shown in 1023 of Fig. 1, the cell sheet production device 100 includes the support unit 10 and a container 20. The support unit 10 is removably held in the container 20.

As shown in 1010 of Fig. 1, the support unit 10 includes a ring member 1, a mesh sheet 2, and a base 3 (holding member). The base 3 has a base body 3a, a cylindrical placement portion 3b, bottom surface abutting portions 3c, and side wall abutting portions 3d.

The base body 3a has a disk shape. The cylindrical placement portion 3b has a bottomless cylindrical shape, and both axial ends of the cylindrical placement portion 3b are open. The cylindrical placement portion 3b is formed to protrude in the vertical direction only from one surface of the base body 3a. The cylindrical placement portion 3b is configured to penetrate the interior of the base body 3a. In other words, upper and lower sides of the base body 3a communicate with each other via the cylindrical placement portion 3b. The mesh sheet 2 is placed on the cylindrical placement portion 3b. The mesh sheet 2 can be observed, with use of a microscope or the like, through the cylindrical placement portion 3b from a side opposite to the cylindrical placement portion 3b with respect to the base body 3a.

The side wall abutting portions 3d each have a rectangular plate shape in plan view from a direction perpendicular to the base body 3a, the rectangular plate shape extending in the horizontal direction from the base body 3a. There are at least two side wall abutting portions 3d which are formed so as to be symmetrical with respect to the base body 3a. In the configuration shown in 1010 of Fig. 1, the number of the side wall abutting portions 3d is four. The side wall abutting portions 3d abut on a side wall of the container 20 at respective distal end portions in the horizontal direction. The side wall abutting portions 3d each serve as a spacer which keeps a constant distance in the horizontal direction between the base body 3a and the side wall of the container 20. Therefore, even in a case where the container 20 shakes in the horizontal direction, the base body 3a is stably kept at an unchanged position in the horizontal direction in the culture medium since the distal end portions of the side wall abutting portions 3d abut on the side wall of the container 20. Note that the base 3 is configured such that all of the side wall abutting portions 3d each form a dimensional clearance with the side wall of container 20 so as to be able to be removed from the container 20.

The bottom surface abutting portions 3c each have a rectangular plate shape, and are each erected with respect to a corresponding one of the side wall abutting portions 3d having the rectangular plate shape. The bottom surface abutting portions 3c each intersect a corresponding one of the side wall abutting portions 3d. In the configuration shown in 1010 of Fig. 1, the bottom surface abutting portions 3c each intersect, in the vertical direction (gravitational direction), a corresponding one of the side wall abutting portions 3d. Then, the bottom surface abutting portions 3c each have a vertical distal end portion which abuts on a bottom surface of the container 20. The bottom surface abutting portions 3c each serve as a spacer which keeps a constant distance in the vertical direction between the base body 3a and the bottom surface of the container 20. Therefore, while the base 3 is held in the container 20, the bottom surface abutting portions 3c abut on the bottom surface of the container 20. This allows the base body 3a to be stably kept at an unchanged position in the vertical direction in the culture medium. Further, the bottom surface abutting portions 3c each intersect a corresponding one of the side wall abutting portions 3d, and are formed so as to protrude from both upper and lower sides of a corresponding one of the side wall abutting portions 3d. Therefore, the position of the base body 3a in the vertical direction is stably kept even in a case where the base body 3 is turned upside down and held in the container 20.

The ring member 1 has a ring shape surrounding an outer periphery of the cylindrical placement portion 3b, and is detachably provided to the base 3. The ring shape of the ring member 1 is not particularly limited, and may be any shape such as a circular ring or a quadrangular ring. In the configuration shown in 1010 of Fig. 1, the ring member 1 has a circular ring shape.

The mesh sheet 2 is placed between the ring member 1 and the cylindrical placement portion 3b. The mesh sheet 2 is held by the base 3, while a peripheral edge of the mesh sheet 2 is sandwiched between an inner surface of the ring member 1 and an outer surface of the cylindrical placement portion 3b. With regard to a difference between an inner diameter of the ring member 1 and an outer diameter of the cylindrical placement portion 3b, in a case where the ring member 1 has a circular ring shape and the cylindrical placement portion 3b has a cylindrical shape, the difference closer to the thickness of the mesh sheet 2 is more preferable from the viewpoint that the mesh sheet 2 is stably held.

Next, the following will discuss the method for producing a cell sheet by using the cell sheet production device 100. First, as shown in 1020 of Fig. 1, the mesh sheet 2 is placed on the cylindrical placement portion 3b of the base 3. Then, while the mesh sheet 2 is kept placed on the cylindrical placement portion 3b, the ring member 1 is mounted to the cylindrical placement portion 3b.

At this time, the ring member 1 is mounted so as to cause the inner surface of the ring member 1 to surround the outer surface of the cylindrical placement portion 3b. Therefore, as shown in 1021 of Fig. 1, the peripheral edge of the mesh sheet 2 is sandwiched between the inner surface of the ring member 1 and the outer surface of the cylindrical placement portion 3b. The support unit 10, which is holding the mesh sheet 2, is thus prepared. In the support unit 10 thus prepared, one of the openings at the both axial ends of the cylindrical placement portion 3b is closed by the mesh sheet 2, and the other one of the openings is opened.

The support unit 10 is then turned upside down so that the mesh sheet 2 is located below the cylindrical placement portion 3b. As a result, in the support unit 10, the mesh sheet 2 and the inner surface of the cylindrical placement portion 3b together form a bottomed cylindrical portion 10a which has the mesh sheet 2 as a bottom surface. As shown in 1022 of Fig. 1, a suspension in which cells 4 are suspended in a culture medium 6 is injected into the bottomed cylindrical portion 10a with use of a Pipetman 5 or the like. At this time, due to the surface tension of the suspension with respect to the inner surface of the cylindrical placement portion 3b and to the mesh sheet 2, the suspension does not pass through the mesh sheet 2 and is held on the mesh sheet 2. Then, the cells 4 in the suspension are caused to adhere to the mesh sheet 2, so that the cells 4 in the suspension are seeded on the mesh sheet 2.

Thereafter, the support unit 10 is held in the container 20. Then, the container 20 is filled with the culture medium 6, and the cells 4 having adhered to the mesh sheet 2 are cultured.

As illustrated in 1030 of Fig. 1, the individual cells 4 adhere to each other while spontaneously extending toward the center of each opening of the mesh sheet 2, so that the opening of the mesh sheet 2 is filled. When the cells 4 which retain proliferative ability are cultured on the mesh sheet 2, the cells 4 grow not only in the horizontal direction but also in the vertical direction with respect to the mesh sheet 2. As a result, it is possible to obtain a cell sheet 200 in which a single cell layer or a plurality of cell layers each made of the cells 4 are formed so as to cover the openings of the mesh sheet 2. It is possible to control the thickness of the cell sheet 200 by controlling an amount of the cells 4 to be seeded on the mesh sheet 2 and/or a growth rate of the cells 4. The cell sheet 200 is strong and easy to handle because the cell sheet 200 is supported by the mesh sheet 2.

Further, the cell sheet 200 is configured to have at least two cell layers each made of the cells 4, and to have the mesh sheet 2 provided between the two cell layers, the mesh sheet 2 being a substrate to which the cells 4 are caused to adhere and on which the cells 4 are cultured. In other words, both surfaces of cell sheet 200 are constituted by the cells 4. Therefore, the cell sheet 200 has no limitation on an attachment surface to one surface of the cell sheet 200, the attachment surface being a surface to be attached to an affected area. Further, as compared with a conventional method for producing a cell sheet by using a dish or a porous membrane as a scaffold, the method for producing a cell sheet by using the cell sheet production device 100 is superior in terms of nutrient supply to the cells 4 and observation of the cells 4. In addition, with use of a sheet which has patterned openings as in the mesh sheet 2, it is possible to obtain cell sheets 200 which have similar structures (in other words, properties) in a highly reproducible manner.

As shown in 1040 of Fig. 1, the container 20 may be a plate in which a plurality of wells 21 are formed. More specifically, the container 20 is a 12-well plate. In this case, the support unit 10 provided with the base 3 is configured to be removably held in a well 21.

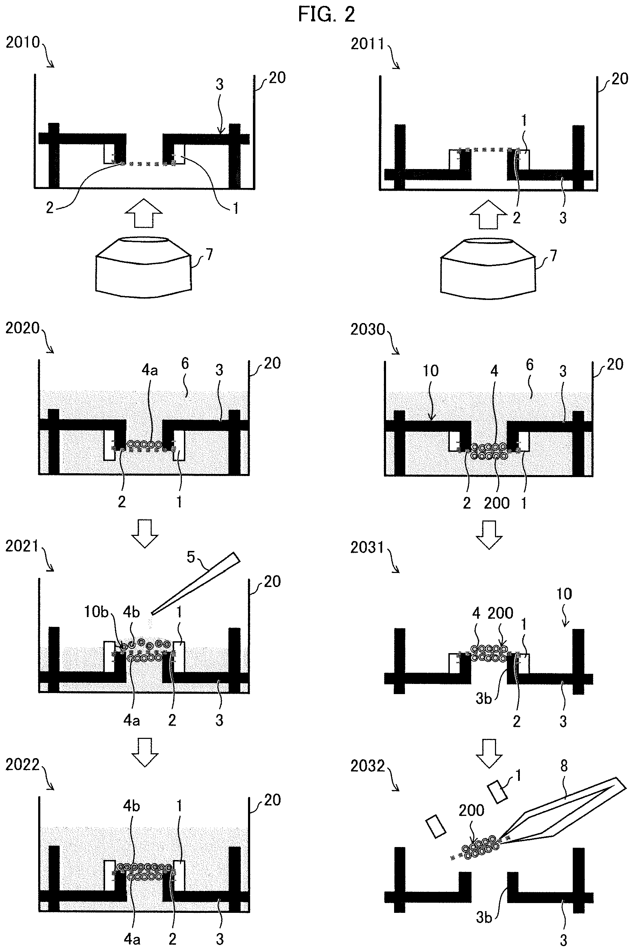

Fig. 2 is a view for describing effects of the cell sheet production device 100. As illustrated in 2010 and 2011 of Fig. 2, the support unit 10 can be used even when the base 3 is turned upside down and held in the container 20. This is because the support unit 10 is removably held in the container 20. When the support unit 10 is reversed, the cell sheet which is formed on the mesh sheet 2 is also reversed. Therefore, it is possible to easily observe, with use of a microscope 7, both surfaces of the cell sheet which has been formed on the mesh sheet 2.

Further, as shown in 2020 to 2022 of Fig. 2, in a case where the cell sheet production device 100 is used, it is possible to prepare a cell sheet having a plurality of types of cell layers. More specifically, it is possible to prepare a cell sheet which has a first cell layer that is made of cells 4a and a second cell layer that is made of cells 4b different in type from the cells 4a. First, as illustrated in 2020 of Fig. 2, the cells 4a are seeded on the mesh sheet 2, and in this state, the support unit 10 is immersed in the culture medium 6. The support unit 10 is then turned upside down as illustrated in 2021 of Fig. 2. At this time, the mesh sheet 2 has, as an upper surface, a surface opposite to the cells 4a. Therefore, the cells 4b can also be seeded on the surface opposite to the cell 4a of the mesh sheet 2, by dropping, from an upper side, a suspension of the cells 4b onto the mesh sheet 2 with use of the Pipetman 5 or the like (see 2022 of Fig. 2). According to the configuration of the cell sheet production device 100, it is possible to additionally seed cells on either one of a front surface and a back surface of the cell sheet. Note that, from the viewpoint of easily seeding cells on both surfaces of the cell sheet, the ring member 1 is preferably arranged to be attached to the base 3 so as to protrude from the cylindrical placement portion 3b, as illustrated in 2020 to 2022 of Fig. 2. According to this configuration, when the support unit 10 is reversed, the bottomed cylindrical portion 10b is formed by the inner surface of the ring member 1 and the mesh sheet 2 so as to have the mesh sheet 2 as a bottom surface. Therefore, the suspension is stably held on the mesh sheet 2 by injecting the suspension of the cells 4b into the bottomed cylindrical portion 10b.

Further, according to the configuration of the cell sheet production device 100, it is possible to easily separate the cell sheet 200 from the support unit 10. As illustrated in 2030 of Fig. 2, the cell sheet 200 which is constituted by the cells 4 is prepared by culturing the cells 4 in the culture medium in the container 20. Thereafter, as shown in 2031 of Fig. 2, the support unit 10 is removed from the container 20. The ring member 1 is then detached from the cylindrical placement portion 3b of the base 3, as illustrated in 2032 of Fig. 2. As a result, the cell sheet 200 which is formed on the mesh sheet 2 can be detached from the cylindrical placement portion 3b of the base 3 by using, for example, tweezers 8. Such a cell sheet 200 which can be easily detached from the cell sheet production device 100 is convenient, for example, in a case where the cell sheet is to be grafted.

### (Variation 1)

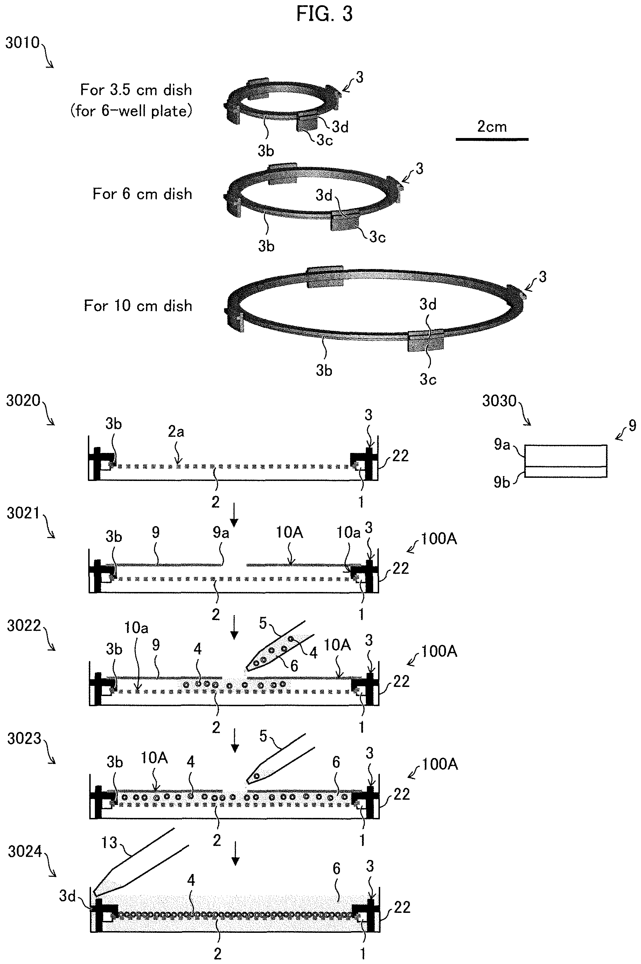

The following will discuss a variation of the cell sheet production device 100. Fig. 3 is a view illustrating, as Variation 1, a configuration of a cell sheet production device 100A and a method for producing a cell sheet by using the cell sheet production device 100A. Fig. 3 includes 3010 which is a perspective view illustrating a configuration example of the base 3 that is provided in the cell sheet production device 100A. Fig. 3 further includes 3020 to 3024 which are views illustrating the method for producing a cell sheet by using the cell sheet production device 100A. Fig. 3 includes 3030 which is a cross sectional view illustrating a configuration of a covering body 9 that is provided in the cell sheet production device 100A. The cell sheet production device 100A is capable of producing a cell sheet having a large area.

As illustrated in 3010 of Fig. 3, the base 3 can be designed for a 3.5 cm diameter dish (for a 6-well plate), a 6 cm diameter dish, and a 10 cm diameter dish. That is, the cell sheet prepared can have a large area, as a result of increasing the diameter of the cylindrical placement portion 3b of the base 3. In accordance with this, the area of the mesh sheet 2 can be increased.

The cell sheet production device 100A according to Variation 1 has a configuration which is suitable for producing a cell sheet having a large area by holding a suspension on the mesh sheet 2 having such a large area. As shown in 3021 to 3024 of Fig. 3, the support unit 10A of the cell sheet production device 100A includes the covering body 9. The covering body 9 is configured to cover one surface 2a of the mesh sheet 2. The covering body 9 is provided with an injection port 9a for injecting the suspension between the mesh sheet 2 and the covering body 9, the suspension containing the cells 4. More specifically, the covering body 9 is arranged so as to cover the surface 2a of the mesh sheet 2, the surface 2a being in contact with the cylindrical placement portion 3b. The mesh sheet 2 and the covering body 9 are spaced apart from each other. The cylindrical placement portion 3b is provided between the mesh sheet 2 and the covering body 9. The cylindrical placement portion 3b keeps a constant distance between the mesh sheet 2 and the covering body 9.

The distance between the mesh sheet 2 and the covering body 9 here is set so that the suspension of the cells 4 can be injected between the mesh sheet 2 and the covering body 9 while coming in contact with both of the mesh sheet 2 and the covering body 9. The distance between the mesh sheet 2 and the covering body 9 is set to preferably 1 mm to 3 mm, more preferably 1 mm to 2 mm, and particularly preferably 1 mm to 1.5 mm.

In other words, the covering body 9 is a member for closing the bottomed cylindrical portion 10a which is formed by the mesh sheet 2 and the cylindrical placement portion 3b. The covering body 9 may have a layered structure which includes at least a first layer 9a and a second layer 9b. The first layer 9a is a layer that is furthest from the mesh sheet 2 and the second layer 9b is a layer that is closest to the mesh sheet 2. In the configuration illustrated in 3030 of Fig. 3, the covering body 9 has a two-layer structure which includes the first layer 9a and the second layer 9b. Examples of a material which constitutes the first layer 9a include a silicone resin. Further, examples of a material which constitutes the second layer 9b include polyethylene terephthalate (PET). Note that the covering body 9 may be a single layer structure which is made of one of the first layer 9a and the second layer 9b.

In the method for producing a cell sheet by using the cell sheet production device 100A, first, the support unit is placed in the dish 22. This support unit is not provided with the covering body 9. Further, the support unit is arranged upside down such that the mesh sheet 2 is located below the cylindrical placement portion 3b. As a result of arranging the support unit 10 in this manner, the bottomed cylindrical portion 10a is formed. The bottomed cylindrical portion 10a is formed by the mesh sheet 2 and the inner surface of the cylindrical placement portion 3b, and the mesh sheet 2 is used as a bottom surface (see 3020 of Fig. 3). Next, the covering body 9 is provided so as to close an open top of the bottomed cylindrical portion 10a. As a result, the support unit 10A is prepared (see 3021 of Fig. 3).

Then, from the injection port 9a, the suspension in which the cells 4 are suspended in the culture medium 6 is injected into the bottomed cylindrical portion 10a with use of a Pipetman 5 or the like (see 3022 of Fig. 3). At this time, the suspension of the cells 4 is injected into the bottomed cylindrical portion 10a while coming in contact with both of the mesh sheet 2 and the covering body 9. Therefore, the surface tension of the suspension with respect to each of covering body 9 and the mesh sheet 2 prevents the suspension from passing and falling through the mesh sheet 2 due to gravity. Therefore, even if the mesh sheet 2 has a large area, the suspension is held on the mesh sheet 2 by the surface tension of the suspension with respect to the covering body 9 (see 3023 of Fig. 3). In this way, the cells 4 in the suspension are caused to adhere to the mesh sheet 2. This allows the cells 4 to be uniformly seeded on the mesh sheet 2 (e.g., the mesh sheet 2 having the area).

Thereafter, the dish 22 is totally filled with the culture medium 6. Then, the cells 4 which have adhered to the mesh sheet 2 are cultured (see 3024 of Fig. 3).

In the above-described method, the suspension of the cells 4 is injected after the covering body 9 has been placed over the bottomed cylindrical portion 10a. However, the covering body 9 may be provided so as to prevent the suspension from dropping off the mesh sheet 2, after the suspension of the cells 4 has been injected into the bottomed cylindrical portion 10a.

### (Variation 2)

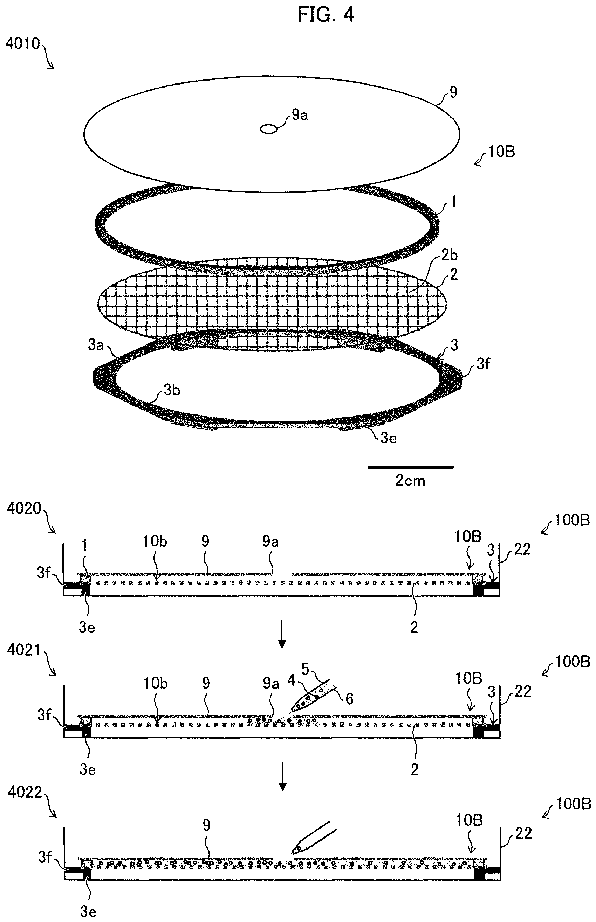

The following will discuss another variation of the cell sheet production device 100. Fig. 4 is a view showing a configuration of a cell sheet production device 100B as Variation 2 and a method for producing a cell sheet by using the cell sheet production device 100B. Fig. 4 includes 4010 which is an exploded perspective view illustrating a configuration of a support unit 10B that is provided in the cell sheet production device 100B. Fig. 4 further includes 4020 to 4022 which are views illustrating the method for producing a cell sheet by using the cell sheet production device 100B.

As in Variation 1, the cell sheet production device 100B is provided with the covering body 9 and can produce a cell sheet having a large area. In the cell sheet production device 100B, the support unit 10B is configured differently from Variation 1. As illustrated in 4010 of Fig. 4, the base 3 of the support unit 10B is not configured such that the cylindrical placement portion 3b protrudes from the base body 3a. The cylindrical placement portion 3b is provided as an opening which is formed in the base body 3a. Bottom surface abutting portions 3e are each provided so as to protrude downward from the base body 3a, and do not protrude upward. Further, side wall abutting portions 3f are each provided so as to extend in the horizontal direction from the base body 3a.

The ring member 1 has an inner diameter that is substantially equal to an inner diameter of the cylindrical placement portion 3b. Therefore, the mesh sheet 2 is sandwiched between a lower surface of the ring member 1 and an upper surface of the base body 3a.

The covering body 9 is provided so as to cover a surface 2b of the mesh sheet 2, the surface 2b coming in contact with the ring member 1. The mesh sheet 2 and the covering body 9 are spaced apart from each other. The ring member 1 is provided between the mesh sheet 2 and the covering body 9. The ring member 1 keeps a constant distance between the mesh sheet 2 and the covering body 9. The covering body 9 is a member for closing the bottomed cylindrical portion 10b which is formed by the mesh sheet 2 and the ring member 1.

In the method for producing a cell sheet by using the cell sheet production device 100B, first, the support unit 10B is placed in the dish 22. The support unit 10B is arranged such that the mesh sheet 2 is located below the ring member 1. As a result of arranging the support unit 10B in this manner, the bottomed cylindrical portion 10b is formed in the support unit 10B. The bottomed cylindrical portion 10b is formed by the mesh sheet 2 and the inner surface of ring member 1, and the mesh sheet 2 is used as a bottom surface (see 4020 of Fig. 4).

Then, from the injection port 9a, the suspension in which the cells 4 are suspended in the culture medium 6 is injected into the bottomed cylindrical portion 10a with use of a Pipetman 5 or the like, so that the cells 4 are seeded on the mesh sheet 2 (see 4021 and 4022 of Fig. 4). Thereafter, the dish 22 is filled with the culture medium 6, and cell culture is performed.

The cell sheet production device 100B is structured such that a cell sheet prepared cannot be easily separated from the support unit 10B. However, the cell sheet can be cut from the support unit 10B, by using scissors or a knife.

The cell sheet prepared by using the cell sheet production device in accordance with the present embodiment allows for supplying nutrients from both of upper and lower surfaces of the cell sheet in the culture medium. In a conventional method according to which a cell sheet is prepared on a dish, it is not possible to supply nutrients from a lower surface of the cell sheet. Accordingly, culture of cells relies only on nutrient supply from an upper surface of the cell sheet. Therefore, in situations where the nutrient supply from the upper surface of the cell sheet is limited, the nutrient supply to the cells inside the cell sheet may be inadequate.

Examples of such a situation where the nutritional supply from the upper side of the cell sheet is limited include a situation where a skin sheet is prepared. In production of a skin sheet, the skin sheet may be produced by forming a keratinocyte layer (epidermal layer) on a fibroblast layer (dermal layer). In this case, mature epidermal cells form a tight junction. This inhibits nutrient supply from a top of the skin sheet. Therefore, it is difficult to prepare, by the conventional method according to which a cell sheet is prepared on a dish, a skin sheet which is constituted by a fibroblast layer and a dermal layer.

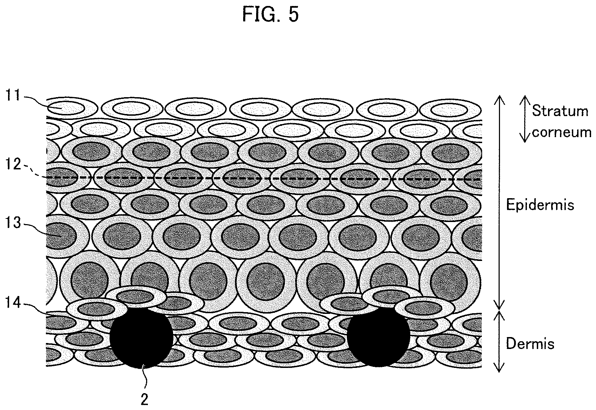

Fig. 5 is a view schematically illustrating a cross-section structure of a skin sheet which is prepared by the cell sheet production device in accordance with the present embodiment. The reference sign 11 indicates dead keratinocytes. The reference sign 12 indicates the above-mentioned tight junction. Meanwhile, the reference sign 13 indicates keratinocytes. The reference sign 14 indicates skin fibroblasts. As illustrated in Fig. 5, the skin sheet which is prepared by using the cell sheet production device in accordance with the present embodiment can be expected to be supplied with nutrients from a layer of the skin fibroblasts 14 at the bottom even in a case where nutrient supply from the keratinocytes 13 at the top decreases. This makes it possible to supply more nutrients to the keratinocytes 13 and the skin fibroblasts 14 which constitute the skin sheet.

Further, known as another conventional technique is a method according to which a skin sheet which includes a plurality of cell layers is prepared on a porous membrane. Since the porous membrane is used, it is considered that nutrients are supplied, to some extent, from a lower surface of the cell sheet. However, it is difficult to directly use, for grafting, the cell sheet which has been prepared by this method. This is because when the skin sheet is grafted on a patient, the skin sheet needs to be grafted such that not a surface of an epidermal layer but a surface of a dermal layer is put in contact with an affected area. It follows that it is impossible to use, for such grafting, the cell sheet in which the dermal layer is sandwiched between the porous membrane and the epidermal layer. In contrast, in the case of the skin sheet which is prepared by using the cell sheet production device in accordance with the present embodiment, cells of not only an epidermal layer surface but also a dermal layer surface are present on a culture medium side, in other words, exposed to the culture medium. Therefore, it is possible to attach the dermal layer surface of the skin sheet to the affected area.

The tight junction 12 is also observed in intestinal tract cells and vascular endothelial cells. Therefore, the cell sheet production device in accordance with the present embodiment can be also used in culturing and using, other than the skin sheet, a cell sheet which includes intestinal tract cells, vascular endothelial cells, or the like cells that form the tight junction 12.

### (Variation 3)

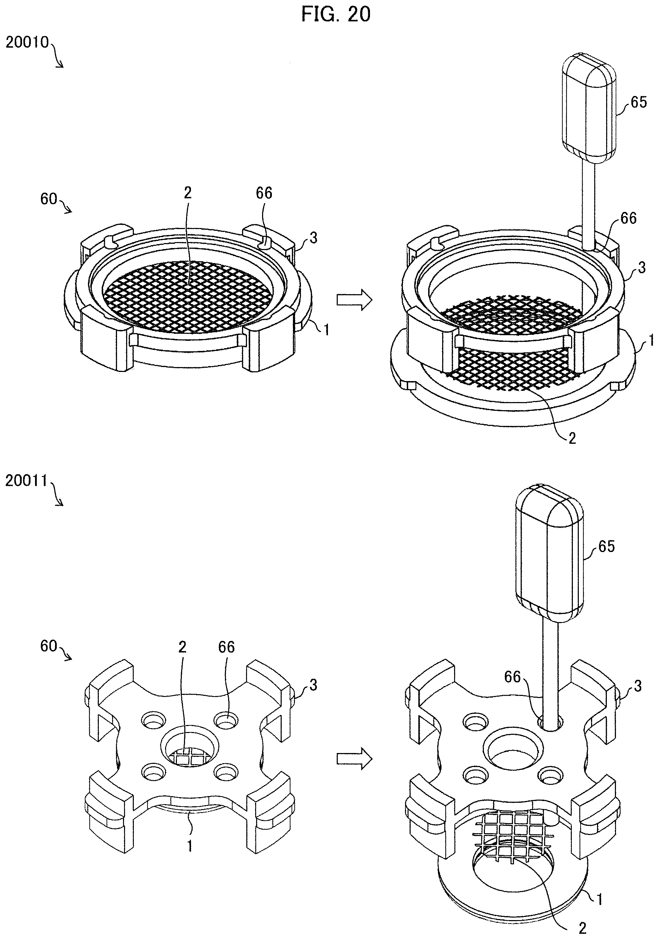

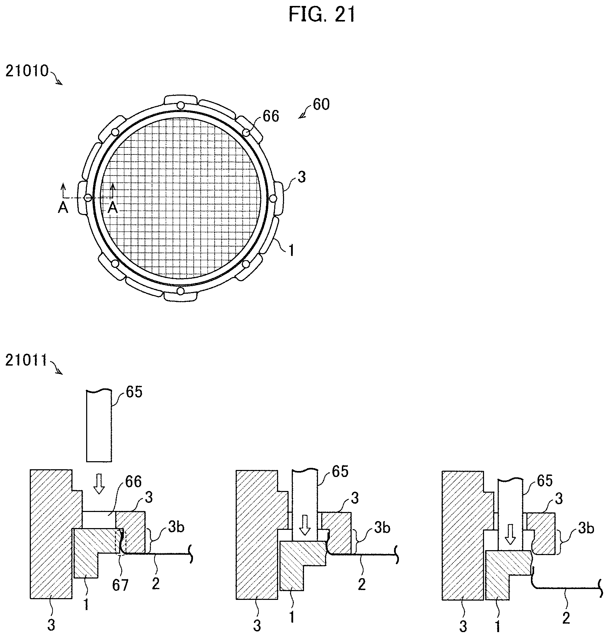

The following will discuss another variation of the cell sheet production device. Figs. 20 and 21 are each a view schematically illustrating a support unit 60 of a cell sheet production device as Variation 3. Note that in Figs. 20 and 21, illustration of a container which is included in the cell sheet production device is omitted.

The cell sheet production device according to the present embodiment is configured such that: at least one of the base 3 (holding member) and the ring member 1 has a locking portion 67 which locks the base 3 and the ring member 1 so that the base 3 and the ring member 1 do not separate from each other; and at least one of the base 3 (holding member) and the ring member 1 has a through hole 66 through which a pushing pin 65 for pushing the other one of the base 3 and the ring member 1 is caused to pass, pushing of the other one of the base 3 and the ring member 1 releasing locking of the base 3 and the ring member 1, so that the base 3 and the ring member 1 are separated from each other. Note that the cell sheet production device according to the present embodiment may or may not be configured to include the pushing pin 65. In a case where the cell sheet production device according to the present embodiment is configured not to include the pushing pin 65, the pushing pin 65 may be prepared as a configuration separate from the cell sheet production device.

The locking portion 67 is not limited to a specific configuration and may have any configuration, provided that locking of the base 3 and the ring member 1 can be released by the pushing pin 65. Examples of the specific configuration of the locking portion 67 include: (i) a frictional force generating area which is formed on at least one surface of the base 3 and the ring member 1 and which generates a large frictional force between the base 3 and the ring member 1; (ii) a combination of a projection that is formed on a surface of the base 3, and a locking projection that is formed on a surface of the ring member 1 and that stops, by locking, movement of the projection on the surface of the base 3 in a push direction; (iii) a combination of a projection that is formed on the surface of the ring member 1, and a locking projection that is formed on the surface of the base 3 and that stops, by locking, movement of the projection on the surface of the ring member 1 in a push direction; and (iv) a combination of (ii) and (iii) described above. The friction generating area can be formed, for example, by attaching a material (e.g., rubber) capable of generating a desired frictional force at a surface of at least one of the base 3 and the ring member 1.

Though the through hole 66 only needs to be formed on one of the base 3 and the ring member 1, it is preferable, from the viewpoint of handleability of the support unit 60, that the through hole 66 be formed on the base 3.

Here, a specific through hole 66 is focused. In a configuration in which this through hole 66 is formed in the base 3, no through hole can be formed in a region of the ring member 1, the region being opposed to the through hole 66. Alternatively, in a configuration in which the through hole 66 is formed in the ring member 1, no through hole can be formed in a region of the base 3, the region being opposed to the through hole 66. The above configuration makes it possible to effectively push, by the pushing pin 65, a component (the base 3 or the ring member 1) in which no through hole is formed, in a case where the pushing pin 65 is caused to pass through the through hole 66.

The through hole 66 is not limited in shape and number. Examples of the shape of the through hole 66 include a cylindrical shape and a prismatic shape. The number of the through hole(s) 66 which is/are formed in each holding member or in each ring member may be an even number or an odd number. From the viewpoint of substantially uniformly pushing the whole of the base 3 or the ring member 1, the number of the through holes 66 is preferably an even number. The number of the through hole(s) 66 may be, for example, 1 to 10 or 1 to 20, but the number of the through hole(s) 66 may be appropriately set on the basis of sizes of the base 3 and the ring member 1.

More specifically, in 20010 and 20011 of Fig. 20, diagrams on the left side of arrows are each a perspective view of the support unit 60. As illustrated in detail in Fig. 21 which will be described later, the cylindrical placement portion 3b, on which the mesh sheet 2 is to be placed, is formed on the base 3, and the peripheral edge of the mesh sheet 2 is sandwiched between the cylindrical placement portion 3b and the ring member 1. Note that the support unit 60 may include a covering body (not illustrated) on the base 3 or the ring member 1.

In 20010 and 20011 of Fig. 20, diagrams on the right side of the arrows are each an exploded perspective view of the support unit 60 after release of locking of the base 3 and the ring member 1 by causing the pushing pin 65 to pass through the through hole 66 and pushing the ring member 1. In each of these views, the through hole 66 is formed in the base 3. Further, no through hole is formed in the region of the ring member 1, the region being opposed to the through hole 66. Therefore, in a case where the pushing pin 65 is caused to pass through the through hole 66, the ring member 1 is effectively pushed by the pushing pin 65 and consequently, locking of the base 3 and the ring member 1 is released. In each of the above views, four through holes 66 are illustratively formed in the base 3. In a case where the pushing pin 65 is caused to pass through each of these through holes 66, the whole of the ring member 1 can be substantially uniformly pushed.

Fig. 21 includes 21010 which is a top view of the support unit 60. Fig. 21 includes 21011 which is a cross sectional view that is taken along the position "A-A" of the support unit 60 and that illustrates changes over time of each component in a case where the pushing pin 65 is caused to pass through the through hole 66. First, as illustrated in a left part of 21011 of Fig. 21, the pushing pin 65 is inserted into the through hole 66 along a direction of an arrow. Then, as illustrated in a middle part of 21011 of Fig. 21, when the pushing pin 65 is caused to pass through the through hole 66, the pushing pin 65 pushes the ring member 1 downward. Finally, the pushing pin 65 further pushes the ring member 1 downward, as illustrated in a right part of 21011 of Fig. 21. This releases locking of the base 3 and the ring member 1, so that the base 3 and the ring member 1 are separated from each other. Consequently, the mesh sheet 2 (specifically, the peripheral edge of the mesh sheet 2) which is sandwiched between the cylindrical placement portion 3b and the ring member 1 is detached.

### 3. Configuration Example 2 of cell sheet production device in accordance with present embodiment

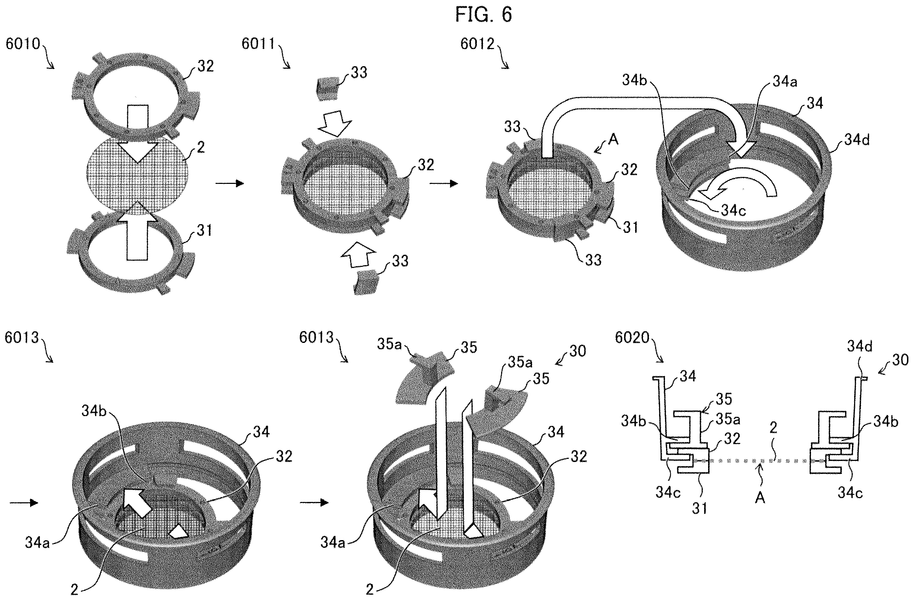

The following will discuss Configuration Example 2 of the cell sheet production device in accordance with the present embodiment. Fig. 6 is a view schematically illustrating a configuration of a support unit 30 which is provided in a cell sheet production device according to Configuration Example 2. Fig. 6 includes 6010 to 6013 which are perspective views illustrating a procedure for assembling the support unit 30. Fig. 6 includes 6020 which is a cross sectional view schematically illustrating a configuration of the support unit 30.

As illustrated in 6010 to 6013 and 6020 of Fig. 6, the support unit 30 includes a mesh sheet 2, a pair of frame bodies 31 and 32, clips 33 (clip member), a holding member 34, and spacers 35. The holding member 34 constitutes a main body of the support unit 30. The mesh sheet 2, the pair of frame bodies 31 and 32, and the clips 33 form a mesh assembly A.

The frame bodies 31 and 32 are members which reinforce the mesh sheet 2 by holding the mesh sheet 2 between the frame bodies 31 and 32. The frame bodies 31 and 32 are arranged such that a peripheral edge of the mesh sheet 2 is sandwiched between the frame bodies 31 and 32. Further, the frame bodies 31 and 32 are detachably provided to the holding member 34.

As illustrated in 6011 of Fig. 6, the clips 33 are each a member for uniting the mesh sheet 2 and the pair of frame bodies 31 and 32 together by pinching the pair of frame bodies 31 and 32. As a result of pinching by the clips 33, the frame bodies 31 and 32 do not separate from each other but are fixed in close contact with each other.

The mesh assembly A is constructed by placing the mesh sheet 2 between the pair of frame bodies 31 and 32 and fixing the frame bodies 31 and 32 by the clips 33 (see 6010 to 6012 of Fig. 6).

The holding member 34 has a bottomless cylindrical shape. The holding member 34 has an inner diameter that is larger than an outer diameter of the pair of frame bodies 31 and 32. The inner wall of the holding member 34 has inner wall portions which are opposed to each other and on each of which a mount portion 34a is formed. The mount portion 34a is for mounting the mesh assembly A. The mount portion 34a has flat plate portions 34b and 34c. The flat plate portions 34b and 34c are provided so as to be spaced apart from each other and so as to project inward in the horizontal direction from the inner wall of the holding member 34. The mesh assembly A is mounted on the holding member 34 by inserting a peripheral edge portion of the mesh assembly A between the flat plate portions 34b and 34c. Further, at an upper end of the holding member 34, a flange 34d protruding in the horizontal direction is formed.

In a state in which the mesh assembly A is mounted on the holding member 34, there is a gap between the mesh assembly A and the flat plate portion 34b (see 6013 of Fig. 6). The spacers 35 are each a member which fills the gap between the mesh assembly A and the flat plate portion 34b. The spacers 35 lock the mesh assembly A so that the mesh assembly A does not move in the mount portion 34a of the holding member 34. Therefore, the mesh assembly A is stably held in the holding member 34.

The spacers 35 are each provided with a tab 35a which protrudes and extends upward. When the mesh assembly A is to be separated from the holding member 34, a user pinches the tab 35a of the spacer 35 and detaches the spacer 35 from the holding member 34. The mesh assembly A is then separated from the holding member 34.

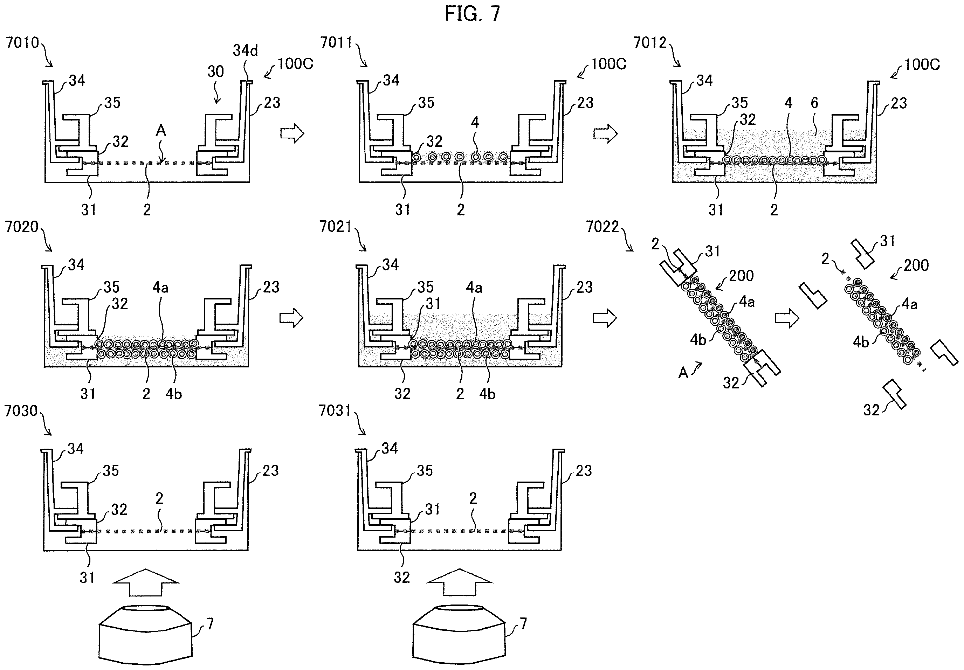

Fig. 7 is a view schematically illustrating a configuration of a cell sheet production device 100C according to Configuration Example 2. Fig. 7 includes 7010 to 7012 which are views illustrating a method for producing a cell sheet by using the cell sheet production device 100C. Fig. 7 also includes 7020 to 7022, 7030 and 7031 which are views illustrating effects of the cell sheet production device 100C.

In the method for producing a cell sheet by using the cell sheet production device 100C, first, the support unit 30 is held in a container 23. At this time, the support unit 30 is arranged so that the mesh assembly A is closest to a bottom surface of the container 23 (see 7010 of Fig. 7).

Further, the flange 34d of the holding member 34 has a lower surface which abuts on an entire upper end surface of the container 23. The outer diameter of the holding member 34 has a dimension which is close to the inner diameter of the container 23. Between an outer peripheral surface of the holding member 34 and an inner side surface of the container 23, a clearance is formed to such an extent that the holding member 34 can be detached from the container 23. This configuration allows the support unit 30 to be held in the container 23 so that the support unit 30 is at a fixed position in the vertical direction and in the horizontal direction in the culture medium.

Next, the cells 4 are seeded on the mesh sheet 2 (see 7011 of Fig. 7). A method of seeding the cells 4 is the same as that in the method with use of the cell sheet production device 100, and therefore, a description thereof will be omitted.

Then, the container 23 is totally filled with the culture medium 6, and the cells 4 having adhered on the mesh sheet 2 are cultured. As a result, a cell sheet 200 is produced.

Next, the following will discuss the effects of the cell sheet production device 100C. As described above, the mesh assembly A is separated from the holding member 34 by detaching the spacer 35 from the holding member 34. The mesh assembly A thus separated can be easily turned upside down and mounted on the holding member 34. Therefore, different types of cells 4a and 4b can be seeded on upper and lower surfaces of the mesh sheet 2, respectively, as illustrated in 7020 and 7021 of Fig. 7. Further, after the cell sheet 200 is formed, the cell sheet 200 can be easily separated from the assembly A. The frame bodies 31 and 32 can be easily separated from each other by detaching the clips 33. Therefore, the cell sheet 200 can be easily taken out by separating the frame bodies 31 and 32. Further, as illustrated in 7030 and 7031 of Fig. 7, both surfaces of the mesh sheet 2 can be observed with use of the microscope 7.

In the cell sheet production device 100C, the support unit 30 is held in the container 23 by abutment of the flange 34d of the holding member 34 on the upper end surface of the container 23. The flange 34d is a portion that does not come in contact with the culture medium 6. Also, the tab 35a of the spacer 35 is a portion that does not come in contact with the culture medium 6. Therefore, when the support unit 30 is to be moved to another container by using tweezers, the spacers 35 are moved upward by the tweezers. This allows the tweezers to move the support unit 30 while the tweezers do not come in contact with the culture medium 6. Further, in a case where the cells 4 are cultured at a position relatively close to a bottom of the container 23, the cells 4 do not come in contact with the support unit 23 even when the support unit 30 is moved.

Note that, even when cells different from the cells 4 are cultured on a bottom surface of the container 23, the cells do not come in contact with the support unit 30. In other words, in the cell sheet production device 100C, when different types of cells are co-cultured respectively (i) on the mesh sheet 2 of the support unit 30 and (ii) on the bottom surface of the container 23, the support unit 30 does not come in contact with the cells which are cultured on the bottom surface of the container 23. Further, since the support unit 30 is inserted from an upper side of the container 23, the position of the support unit 30 is stabilized with respect to the container 23.

### 4. Configuration Example 3 of cell sheet production device in accordance with present embodiment

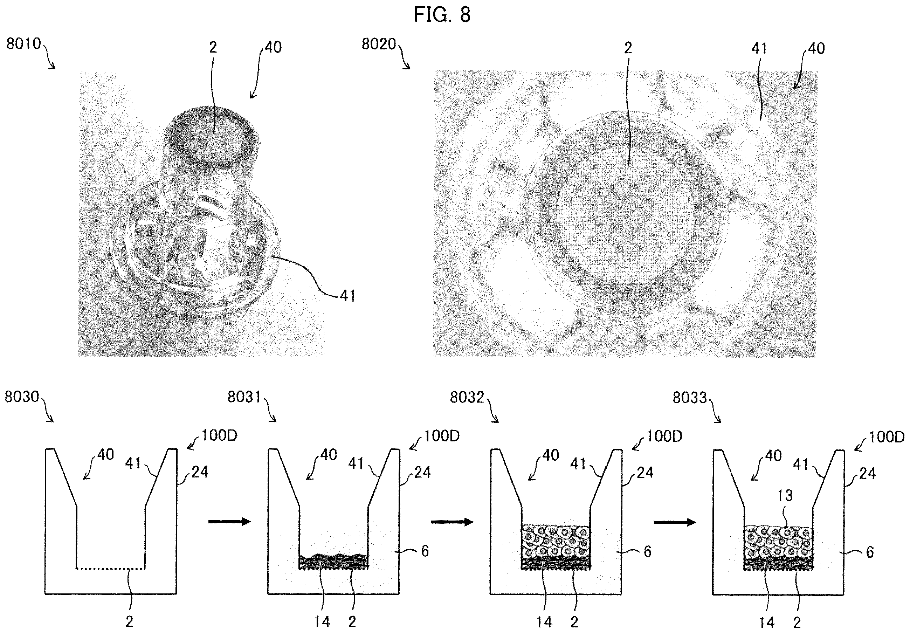

The following will discuss Configuration Example 3 of the cell sheet production device in accordance with the present embodiment. Fig. 8 is a view illustrating a configuration of a cell sheet production device 100D according to Configuration Example 3. Fig. 8 includes 8010 and 8020 each of which shows an image of a configuration example of a support unit 40 that is provided in the cell sheet production device 100D. Fig. 8 further includes 8030 to 8033 which are views illustrating a method for producing a cell sheet by using the cell sheet production device 100D.

The support unit 40 is in the form of a column which is removably held in a well 24 (container). The support unit 40 includes a mesh sheet 2 and a column body 41. The column body 41 has a lower surface which is made of the mesh sheet 2. Further, the column body 41 is held in the well 24 by placing a flange portion of the column body 41 on the well 24. This allows the column body 41 to be at a fixed position in the vertical direction and in the horizontal direction in the culture medium 6 (see 8030 of Fig. 8). Note that the support unit 40 may be prepared, for example, by removing a porous membrane from a column of a commercially available Transwell (registered trademark) and attaching the mesh sheet 2 to the column.

Use of the cell sheet production device 100D makes it easy to subject, to liquid phase-gas phase culture, a cell sheet that has been prepared. For example, in production of a skin sheet, first, as illustrated in 8031 of Fig. 8, skin fibroblasts 14 are seeded on the mesh sheet 2 and cultured in the culture medium 6. Next, as illustrated in 8032 of Fig. 8, keratinocytes 13 are further seeded on a layer consisting of grown skin fibroblasts 14 and are cultured in the culture medium 6, so that a skin sheet is prepared. As illustrated in 8033 of Fig. 8, the skin sheet thus prepared can be cultured in a condition in which the skin fibroblasts 14 are in contact with the culture medium while some of the keratinocytes 13 are in contact with gas, in other words, liquid phase-gas phase culture can be performed.

### 5. Configuration Example 4 of cell sheet production device in accordance with present embodiment

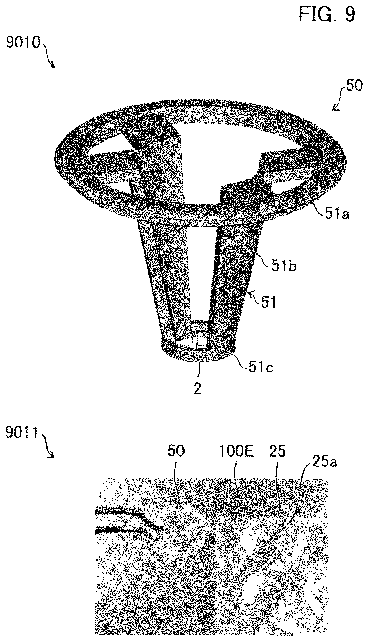

The following will discuss Configuration Example 4 of the cell sheet production device in accordance with the present embodiment. Fig. 9 is a view showing a configuration of a cell sheet production device 100E according to Configuration Example 3. Fig. 9 includes 9010 which is a perspective view illustrating a configuration of a support unit 50 which is provided in the cell sheet production device 100E. Fig. 9 also includes 9011 which shows an image of the configuration of the cell sheet production device 100E.

As shown in 9011 of Fig. 9, the cell sheet production device 100E uses, as a container, a well plate 26 in which a plurality of well 25a are formed. In the configuration shown in 9011 of Fig. 9, the well plate 26 is a 12-well plate. The support unit 50 is removably held in such a well plate 26. The support unit 50 includes a mesh sheet 2 and a column body 51.

The column body 51 has a first annular portion 51a, a foot portion 51b, and a second annular portion 51c. The foot portion 51b is a portion that connects between the first annular portion 51a and the second annular portion 51c. The first annular portion 51a and the second annular portion 51c are arranged so that respective center axes of the first and second annular portions 51a and 51c can coincide with each other.

The first annular portion 51a has an outer diameter that is larger than an inner diameter of the well 25a of the well plate 25. The second annular portion 51c has an inner diameter that is smaller than an inner diameter of the first annular portion 51a. The second annular portion 51c has a bottom portion which is closed by the mesh sheet 2.

In the cell sheet production device 100E, the first annular portion 51a of the column body 51 is placed on the well 25a. The column body 51 is thus held in the well 25a so that the column body 51 is at a fixed position in the vertical direction and in the horizontal direction in the culture medium 6. Further, into a space formed by an inner surface of the second annular portion 51c, an inner surface of the foot portion 51b, and the mesh sheet 2, a suspension of cells is injected.

In the cell sheet production device 100E, the first annular portion 51a of the column body 51 is placed on the well 25a, so that the support unit 50 is held in the well 25a. The first annular portion 51a is a portion that does not come in contact with the culture medium 6. Therefore, when the support unit 50 is to be moved into another well 25a by using tweezers, the first annular portion 51a is pinched by the tweezers. This allows the tweezers to move the support unit 50 while the tweezers do not come in contact with the culture medium 6. Further, in a case where cells are cultured at a position relatively close to a bottom of the well 25a, the cells 4 do not come in contact with the well 25a even when the support unit 50 is moved. Note that in the cell sheet production device 100E, when different types of cells are co-cultured respectively (i) on the mesh sheet 2 of the support unit 50 and (ii) on a bottom surface of the well 25a, the support unit 50 does not come in contact with the cells which are cultured on the bottom surface of the well 25a.

Further, in the cell sheet production device 100E, the mesh sheet 2 is held at a stable position by the first annular portion 51a and the foot portion 51b. Therefore, the cell sheet production device 100E allows the support unit 50 to be held at a more stable position in the vertical direction and in the horizontal direction in the well 25a.

Aspects of the present invention can also be expressed as follows:
In order to solve the above problems, a cell sheet production device in accordance with an aspect of the present invention includes: a container configured to contain a culture medium for culturing cells; and a support unit configured to be removably held in the container, the support unit including: a mesh sheet that is a substrate to which the cells are caused to adhere and on which the cells are cultured; and a holding member which holds the mesh sheet such that the mesh sheet floats from a bottom surface of the container, the support unit being held in the container such that the support unit is at a fixed position in vertical and horizontal directions in the culture medium.

In the above configuration, the support unit is removably held in the container. Accordingly, a user can easily reverse the support unit, by taking out the support unit from the container, reversing the support member, and then putting the support member back in the container. When the support unit is reversed, the cell sheet is also reversed, the cell sheet having been formed on the mesh sheet which is held by the holding member of the support unit. Therefore, the above configuration makes it possible to easily observe, with use of a microscope, both surfaces of the cell sheet which has been formed on the mesh sheet.

Further, the support unit is held in the container such that the support unit is at a fixed position in the vertical and horizontal directions in the culture medium. Accordingly, during observation of the cell sheet with use of the microscope, no change occurs in the position of the support unit, in other words, the position of the cell sheet which has been formed on the mesh sheet. Therefore, the above configuration allows for precise observation of the cell sheet.

The cell sheet production device in accordance with an aspect of the present invention is preferably configured such that the holding member detachably holds the mesh sheet.

The above configuration makes it possible to easily separate, from the support unit, the cell sheet which has been formed on the mesh sheet.

The cell sheet production device in accordance with an aspect of the present invention is preferably configured such that: the holding member includes a cylindrical placement portion on which the mesh sheet is placed; the support unit includes a ring member which has a shape surrounding an outer periphery of the cylindrical placement portion and which is detachably provided to the holding member; and the mesh sheet has a peripheral edge sandwiched between the cylindrical placement portion and the ring member.

In the above configuration, the peripheral edge of the mesh sheet is sandwiched between the cylindrical placement portion and the ring member by attachment to the ring member and the holding member. This allows the mesh sheet to be stably placed on the cylindrical placement portion. Meanwhile, the peripheral edge of the mesh sheet is released from being sandwiched between the cylindrical placement portion and the ring member by detachment from the ring member and the holding member. This allows the mesh sheet placed on the cylindrical placement portion to be easily detached.

The cell sheet production device in accordance with an aspect of the present invention is preferably configured such that: at least one of the holding member and the ring member includes a locking portion which locks the holding member and the ring member so that the holding member and the ring member do not separate from each other; at least one of the holding member and the ring member includes a through hole through which a pushing pin for pushing the other one of the holding member and the ring member is caused to pass; and pushing the other one of the holding member and the ring member releases locking of the holding member and the ring member, so that the holding member and the ring member are separated from each other.

In the above configuration, locking of the holding member and the ring member is released by inserting the pushing pin into the through hole and pushing the holding member or the ring member by the pushing pin. This makes it possible to easily detach the mesh sheet which is placed on the cylindrical placement portion.

The cell sheet production device in accordance with an aspect of the present invention is preferably configured such that the support unit includes: a pair of frame bodies which are detachably provided to the holding member such that a peripheral edge of the mesh sheet is sandwiched between the pair of frame bodies; and a clip member which unites the mesh sheet and the pair of frame bodies by clamping the pair of frame bodies.

In the above configuration, the pair of frame bodies provided so as to hold the peripheral edge of the mesh sheet between the frame bodies is detachably provided to the holding member. This makes it possible to easily detach the mesh sheet which is held by the pair of frame bodies, by separating the pair of frame bodies from the holding member.

The cell sheet production device in accordance with an aspect of the present invention is preferably configured such that the holding member has a larger specific gravity than the culture medium.

In the above configuration, the holding member has a larger specific gravity than the culture medium. This keeps the holding member and the cell sheet, which is formed on the mesh sheet held by the holding member, at respective stable positions in the vertical and horizontal directions in the culture medium.

The cell sheet production device in accordance with an aspect of the present invention is preferably configured such that: the support unit includes a covering body which covers one surface of the mesh sheet, the covering body being provided with an injection port for injecting a suspension between the mesh sheet and the covering body, the suspension containing the cells; and a distance between the mesh sheet and the covering body is set so that while coming in contact with both of the mesh sheet and the covering body, the suspension is injected between the mesh sheet and the covering body.

In the above configuration, the distance between the mesh sheet and the covering body is set so that while coming in contact with both of the mesh sheet and the covering body, the suspension is injected between the mesh sheet and the covering body. In this case, the suspension which contains the cells can be laterally spread while the suspension is prevented from passing through the mesh sheet and moving down to below the mesh sheet. Therefore, even when the mesh sheet has an increased area, the suspension which contains the cells can be uniformly spread on the mesh sheet. This makes it possible to easily perform cell culture with use of a large-area mesh sheet.

The cell sheet production device in accordance with an aspect of the present invention is preferably configured such that the mesh sheet has openings having a shape elongated in one direction.

In a case where, as in the above configuration, the openings of the mesh sheet each have the shape which is elongated in one direction, the openings each have a large wall surface which extends in one direction. There are many cells that adhere to the wall surface and grow, and the cells each grow in a similar orientation. Therefore, according to the above configuration, it is possible to control the orientation of growing cells so that the orientation is in the direction of elongation of the openings.

A cell sheet in accordance with an aspect of the present invention includes: at least two cell layers; and a mesh sheet between the two cell layers, the mesh sheet being a substrate to which cells are caused to adhere and on which the cells are cultured.

The above configuration makes it possible to obtain a sufficient thickness of the cell sheet and to improve strength of the cell sheet. Further, in the above configuration, it is possible to provide a cell sheet which has a plurality of kinds of cell layers.

### Examples

### [Example 1: Cell culture with use of cell sheet production device according to Configuration Example 1]

### <Preparation of support unit 10>

A design drawing of a base 3, which is to be held in a 12-well plate, was prepared. Then, the base 3 was prepared, on the basis of the design drawing, by using a 3D printer AGILISTA-3200 (Keyence). A resultant 3D printed object (the base 3) was coated by using a lab coater (PDS-2010) (Parylene Japan), with use of parylene (DPXC, CAS No. 28804-46-8) (Parylene Japan), which is excellent in biocompatibility. For a mesh sheet 2, a polyester micromesh sheet (AG00Z3N9) (Amaike Textile) was used.

Further, a ring member 1 was prepared with use of a 2-mm plate which was made of polydimethylsiloxane (PDMS) (trade name: SILPOT 184, Toray Dow Corning). The 2-mm PDMS plate was prepared as follows. First, a PDMS solution, which had been obtained by mixing a main agent and a curing agent at a ratio of 10:1, was poured into a dish to a thickness of 2 mm. A completed PDMS plate (2 mm in thickness) was cut by two biopsy trepans (Kai Industries) which had respective diameters of 8 mm and 6 mm, so that a ring (having an outer diameter of 8 mm and an inner diameter of 6 mm) was prepared.

The ring member 1, the mesh sheet 2, and the base 3, which had been prepared as described above, were washed with 70% ethanol and air-dried, and then assembled in a clean bench. A support unit 10 was thus completed. After the support unit 10 thus completed was subjected to UV-treatment for 30 minutes, the support unit 10 was held in each well of the 12-well plate.

### <Cell culture conditions>

Tig-1-20 cells (Product No. JCRB0501) were obtained from JCRB cell Bank (Osaka). The Tig-1-20 cells are human lung-derived normal fibroblasts. The Tig-1-20 cells were cultured with use of Dulbecco's Modified Eagle's Medium (DMEM) (GIBCO) which contained 10% fetal bovine serum (FBS) (GIBCO) and 100 units/mL of penicillin and 100 µg/mL of streptomycin (GIBCO). The Tig-1-20 cells were cultured in an incubator under conditions of 37°C and 5% carbon dioxide (CO₂).

HepG2 cells (Product Number RCB1886) were obtained from RIKEN BioResource Research Center (RIKEN BRC). The HepG2 cells were cultured with use of Dulbecco's Modified Eagle's Medium (DMEM) (GIBCO) which contained 10% fetal bovine serum (FBS) (GIBCO) and 100 units/mL of penicillin and 100 µg/mL of streptomycin (GIBCO). The (Product Number RCB1886) RIKEN BioResource Research Center were cultured in an incubator under conditions of 37°C and 5% carbon dioxide (CO₂).

Human mesenchymal stem cells from adipose tissue were purchased from PromoCell GmbH. The human mesenchymal stem cells from adipose tissue were cultured with use of Mesenchymal Stem Cell Growth Medium 2 (PromoCell GmbH). The human mesenchymal stem cells from adipose tissue were cultured in an incubator under conditions of 37°C and 5% carbon dioxide (CO₂).

### <Cell seeding and microscope observation>

On the mesh sheet 2 (circular region having a diameter of 4 mm) of the support unit 10, 0.5 × 10⁵ (50 µL of 1 × 10⁶ cells/mL of) Tig-1-20 cells, HepG2 cells, or mesenchymal stem cells were seeded. After 5 hours from seeding, 3 mL of a culture medium was added to each well which held the support unit 10. The culture medium was changed once every 3 days (3 mL/well). The cells were photographed with use of a digital microscope or an inverted phase contrast microscope.

A Tig-1-20 cell sheet, a HepG2 cell sheet, and a mesenchymal stem cell sheet, which were obtained by 16-day culturing, were analyzed with use of an optical coherence tomography system Cell3imager Estier (SCREEN Holdings).

For the Tig-1-20 cells, the mesh sheet 2 was coated with a collagen solution prior to cell seeding. More specifically, 5 mg/mL of a collagen acidic solution I-AC (KOKEN) was diluted 15-fold with a 1mM HCl solution, and 50 µL of a diluted collagen solution thus obtained was put on the mesh sheet 2. Then, after the diluted collagen solution was left to stand still for 30 minutes at 37°C, the diluted collagen solution was removed. Thereafter, the mesh sheet 2 was washed twice with PBS (-) and stored in an incubator at 37°C until cell seeding.

### <Results>

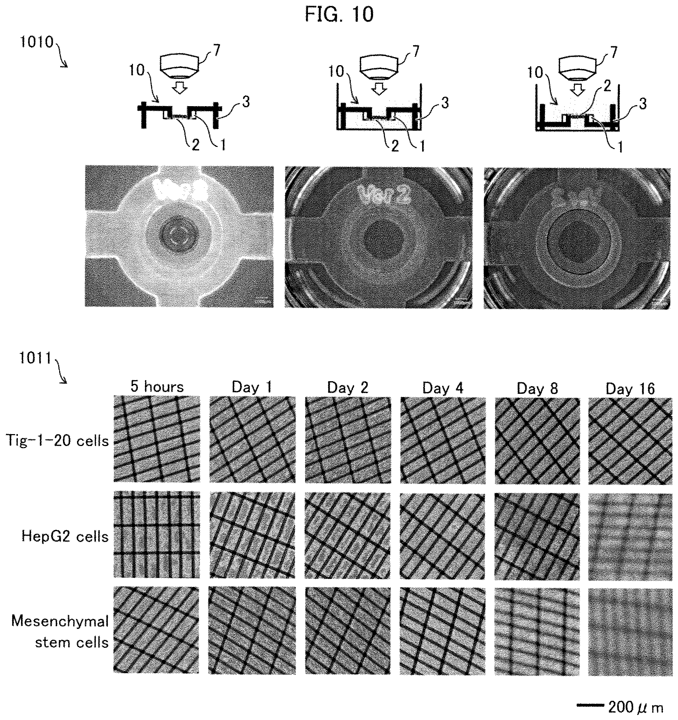

Fig. 10 includes 1010 which shows microscope images of the support unit 10 one day after the cell seeding. On the mesh sheet 2, 50 µL of a cell suspension was stably held (left diagrams). The culture medium could be added to each well without causing any trouble such as formation of bubbles in a gap (center diagrams). Further, the support unit 10 could be turned upside down (right diagrams).

It was confirmed that the Tig-1-20 cells, the HepG2 cells, and the mesenchymal stem cells could be cultured on the mesh sheet 2 (1011 of Fig. 10). Since adhesion of the Tig-1-20 cells to the mesh sheet 2 was weak, the mesh sheet 2 was coated with the coating solution. It was confirmed that coating with the collagen solution caused the cell sheet to stick to the mesh even when the culture medium was added after 5 hours.

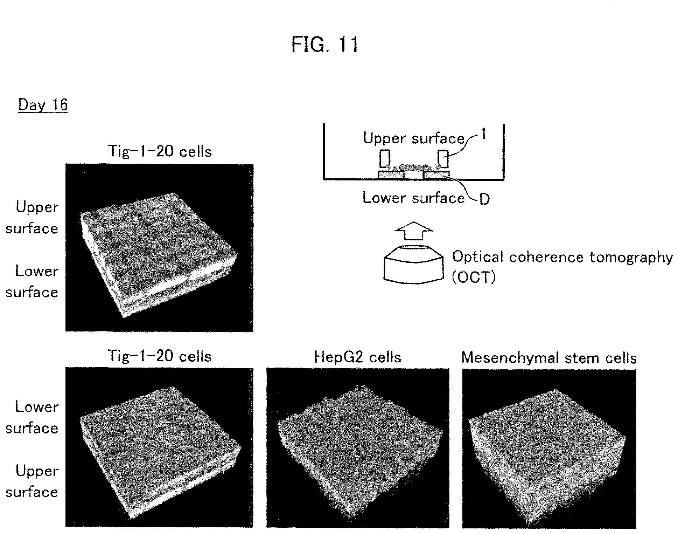

Further, Fig. 11 shows results of analyzing, with use of the optical coherence tomography system, the Tig-1-20 cell sheet, the HepG2 cell sheet, and the mesenchymal stem cell sheet which were obtained by 16-day culturing. It was clear from Fig. 11 that a surface of the Tig-1-20 cell sheet and a surface of the mesenchymal stem cell sheet were flat, whereas a surface of the HepG2 cell sheet was not flat. In analysis with use of the optical coherence tomography system from one side, the HepG2 cell sheet and the mesenchymal stem cell sheet could be analyzed neatly to a plane of the mesh sheet 2. Since the Tig-1-20 cell sheet was thinner than the HepG2 cell sheet and the mesenchymal stem cell sheet, surfaces of the Tig-1-20 cell sheet could be analyzed by observation from one side (Fig. 11).

### [Example 2: Preparation of cell sheet constituted by three kinds of cells, with use of cell sheet production device according to Configuration Example 1]

### <Cell culture conditions>

Cell culture conditions are the same as those in Example 1.

### <Cell seeding on mesh sheet 2 of support unit 10>

A mesh sheet 2 of a support unit 10 (for a 12-well plate) was coated with collagen. Thereafter, on the mesh sheet 2, 4 × 10⁵ (40 µL of 1 × 10⁷ cells/mL of) Tig-1-20 cells were seeded. The Tig-1-20 cells were cells which had been stained with a CellBrite Green Cytoplasmic Membrane-Labeling Kit (diluted 80-fold; Biotium, Inc.). After 5 hours, 3 mL of an FBS-containing DMEM was added to wells. Two days later, from above the Tig-1-20 cells, 4 × 10⁵ (25 µL of 1.6 × 10⁷ cells/mL of) mesenchymal stem cells (MSCs) were seeded. The MSCs were cells which had been stained with a CellBrite Red Cytoplasmic Membrane-Labeling diluted 120-fold; Biotium, Inc.) Kit were seeded. The culture medium was then replaced with a culture medium for the mesenchymal stem cells. The next day, the support unit 10 was turned upside down (inverted). Then, from above the Tig-1-20 cells, 4 × 10⁵ (25 µL of 1.6 × 10⁷ cells/mL of) HepG2 cells were seeded. The HepG2 cells were cells which had been stained with 0.025 mg/mL DiI (1,1'-dioctadecyl-3,3,3',3'-tetramethylindocarbocyanine perchlorate). The next day, the above cells were washed with PBS (-). Further, while immersed in 4% PFA, the cells were left to stand still for 50 minutes at room temperature. After washed with PBS (-), a cell sheet was fixed together with a mounting agent VECTASHIELD (VECTOR), between a cover glass and a slide glass. Specifically, in order to prevent collapse of the cell sheet due to the cover glass, three approximately-100-µm-thick Kapton tape pieces stacked on top of each other were placed between the cover glass and the slide glass. The cell sheet thus fixed was analyzed with use of a confocal microscope LSM 800 (ZEISS).

### <Results>

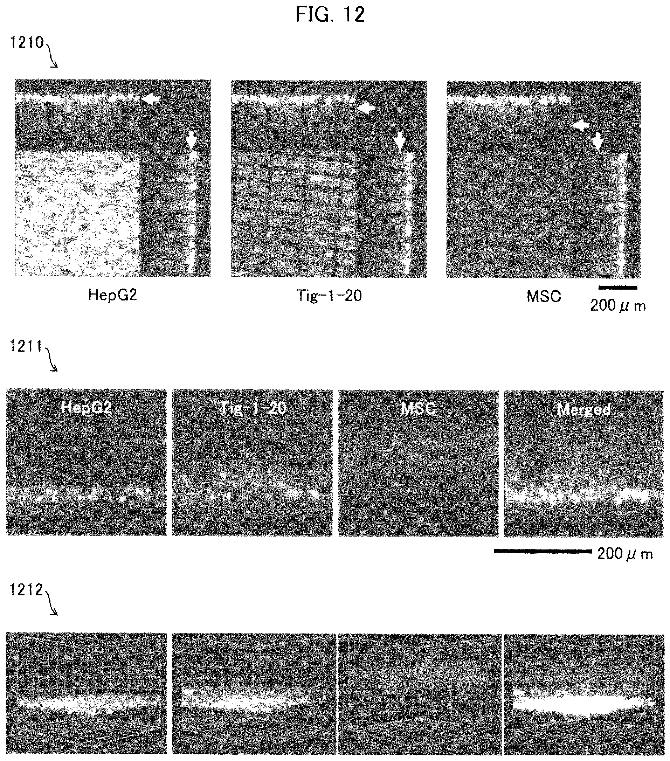

Fig. 12 includes 1210 to 1212 which show results of Example 2. As shown in 1210 to 1212 of Fig. 12, it was possible to prepare a mesenchymal stem cell sheet on the Tig-1-20 cell sheet. In addition, it was also possible to prepare a HepG2 cell sheet under the Tig-1-20 cell sheet. The cell sheet had a total thickness of approximately 140 µm to 150 µm. This result showed that (i) cells could be seeded on both upper and lower sides of the support unit 10, and (ii) a cell sheet including a plurality of kinds of cells could be prepared.

### [Example 3: Culture of human skin fibroblasts with use of cell sheet production device according to Configuration Example 2]

### <Preparation of support unit 30>

Various members, except for a mesh sheet 2, of a support unit 30 were prepared by using a 3D printer AGILISTA-3200. The various members thus prepared were coated with parylene (DPXC, CAS No. 28804-46-8). For the mesh sheet 2, a polyester micromesh sheet (AG00Z3N9) (Amaike Textile) was used. As a control group, another support unit 30 was also prepared by mounting, in place of the mesh sheet 2, a porous membrane (Transwell polycarbonate membrane, pore size of 0.4 µm, Corning Inc.) which had been used for a Transwell (registered trademark).

### <Cell culture conditions>

Normal human adult skin fibroblasts (normal human dermal fibroblasts: NHDFs) were purchased from PromoCell GmbH, and cultured with use of Mesenchymal Stem Cell Growth Medium 2 (PromoCell GmbH). The normal human adult skin fibroblasts were cultured in an incubator under conditions of 37°C and 5% carbon dioxide (CO₂).

### <Cell seeding and microscope observation>

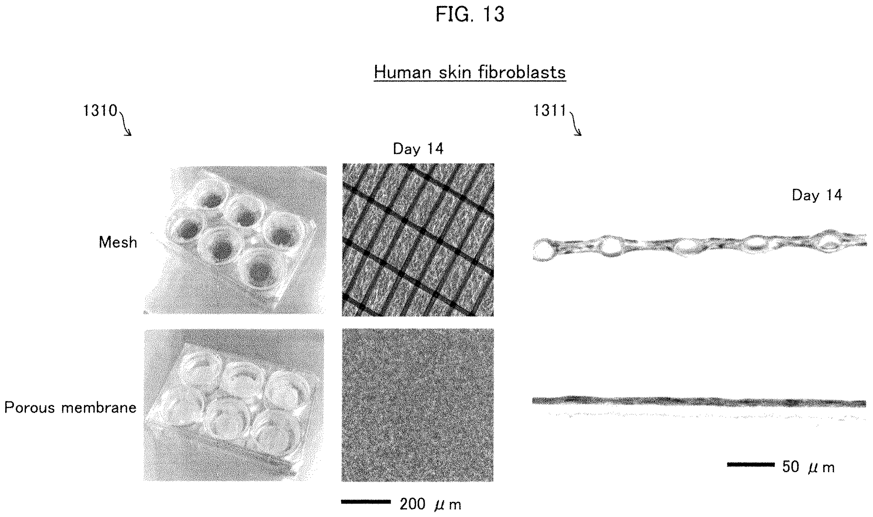

On the mesh sheet 2 of the support unit 30, 1.65 × 10⁶ (550 µL/sheet of 3 × 0⁶ cells/mL of) NHDFs were seeded. Approximately 18 hours later, a culture medium was added. The culture medium was changed once every 3 days (6 mL/well), and culture was performed for 14 days. Inverted phase contrast microscope images of the cells were taken (Fig. 11A). Further, an HE-stained section was prepared (Shin Soshiki Kagaku), and a cross sectional view of the cell sheet was photographed by using BZ-X710 All-in-one (Keyence) (1311 in Fig. 13).

### <Results>

Fig. 13 includes 1310 and 1311 which show results of the above observation. The NHDFs were present so as to fill openings of the mesh sheet 2 and to cover threads of the mesh sheet 2. Each of both of the cell sheet which had been prepared with the mesh sheet 2 and the cell sheet which had been prepared with use of the porous membrane had a smooth surface of the cell sheet. The cell sheet which had been prepared with use of the mesh sheet 2 had exposed cells on a lower surface of the cell sheet. In this regard, the cell sheet which had been prepared with use of the mesh sheet 2 was different from the cell sheet which had been prepared with use of the porous membrane. Therefore, the cell sheet which had been prepared with use of the mesh sheet 2 was considered to be excellent not only in nutrients supply and cell observation but also in attachment of the cell sheet in medical grafting.

### [Example 4: Tig-1-20 cell culture with use of cell sheet production device according to Configuration Example 2]

### <Preparation of support unit 30>

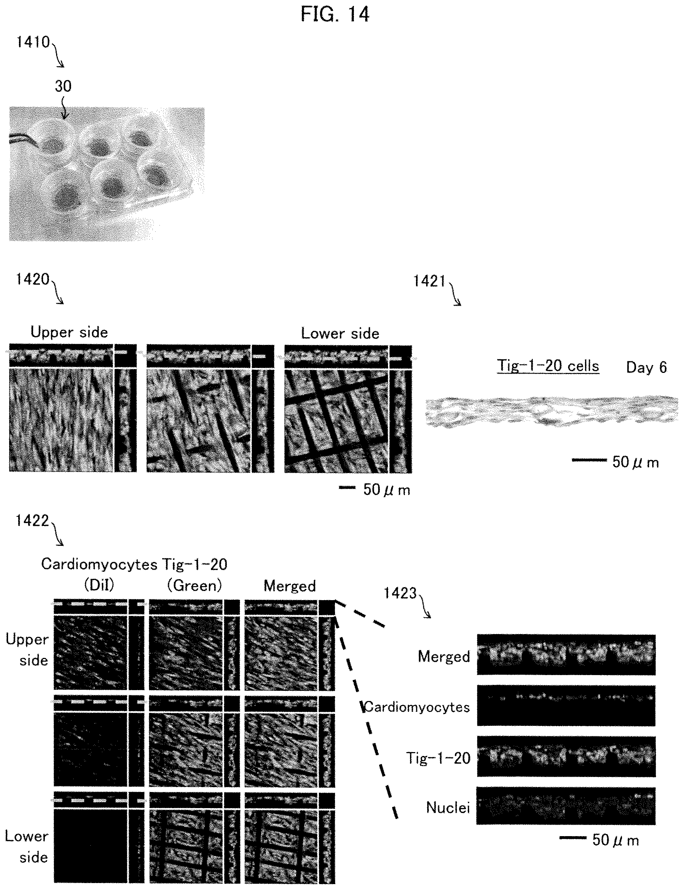

A support unit 30 of Example 4 is prepared as in Example 3. However, the support unit 30 of Example 4 partially differs from that of Example 3, that is, differs only in structure of the holding member 34 from that of Example 3. In Example 4, the holding member 34 has no empty space which facilitates flow of a culture medium from an outer side to an inner side (1410 of Fig. 14).

In addition, culture conditions (composition of the culture medium) of Tig-1-20 cells were the same as those of Example 1. However, in Example 4, the mesh sheet 2 is not coated.

### <Seeding and microscope observation of Tig-1-20 cells>

On the mesh sheet 2 of the support unit 30 which had been prepared in <Preparation of support unit 30>, 4 × 10⁶ (500 µL/mesh of 8 × 10⁶ cells/mL of) Tig-1-20 cells were seeded. The Tig-1-20 cells were cells which had been or had not been stained with a CellBrite Green Cytoplasmic Membrane-Labeling Kit (diluted 100-fold; Biotium, Inc.). Then, 17 hours later, 6 mL of the culture medium was added to each well. Thereafter, three days later, the culture medium was changed once. On sixth day from the start of culture, a cell sheet prepared was washed, and cells were fixed with 4% paraformaldehyde (PFA). Then, after washed, the cells were fixed in a mounting agent VECTA which contained 4',6-diamidino-2-phenylindole (DAPI). With use of a confocal microscope LSM 800 (ZEISS), the cell sheet thus fixed was analyzed. On the other hand, an HE-stained section of a non-fluorescently-stained cell sheet was prepared (Shin Soshiki Kagaku), the non-fluorescently-stained cell sheet having been fixed with 4% PFA and 1% formaldehyde. Then, a cross sectional view of the cell sheet was photographed by using BZ-X710 All-in-one (Keyence).

### <Seeding and microscope observation of human iPS cell-derived cardiomyocytes>

In order to additionally seed human iPS cell-derived cardiomyocytes from above a Tig-1-20 cell sheet (obtained by 6-day culturing) that had not been used for analysis in the above experiment, the culture medium in a well was replaced with 4 ml of MiraCell CM Culture Medium. Then, 1.8 × 10⁵ human iPS cell-derived cardiomyocytes stained with 0.025 mg/mL DiI (1, 1'-dioctadecyl-3,3,3',3'-tetramethylindocarbocyanine perchlorate) were seeded. Approximately 16 hours later, 2 mL of the culture medium was added. Thereafter, the human iPS cell-derived myocardial cells were cultured with the Tig-1-20 cells for 3 days. Then, the cells thus cultured were fixed as described above, and analyzed with use of a confocal microscope.

### <Results>

Fig. 14 includes 1420 to 1423 which show results of the above analysis. As shown in 1420 of Fig. 14, the Tig-1-20 cells were oriented in accordance with the shape of openings of the micromesh sheet. That is, the Tig-1-20 cells were oriented substantially in parallel to long sides of the openings that were rectangular. Also, cells located outside a plane of the micromesh sheet, that is, cells located in a plane above or below the micromesh sheet, were oriented in a direction in which cells in the plane of the micromesh sheet were oriented. The cell sheet had a thickness of approximately 50 µm (1421 of Fig. 14). Further, as a result of additionally seeding the human iPS cell-derived cardiomyocytes from above the Tig-1-20 cell sheet, the cardiomyocytes were also oriented in the direction in which the Tig-1-20 cells were oriented. It was clear from these results that in some cases, orientation of a three dimensional cell sheet may be controlled by cell culture with use of a macro-mesh sheet (1422 and 1423 of Fig. 14).

### [Example 5: Tig-1-20 cell culture with use of cell sheet production device according to Variation 2]

### <Method>

### <Preparation of support unit 10B and cell culture conditions>

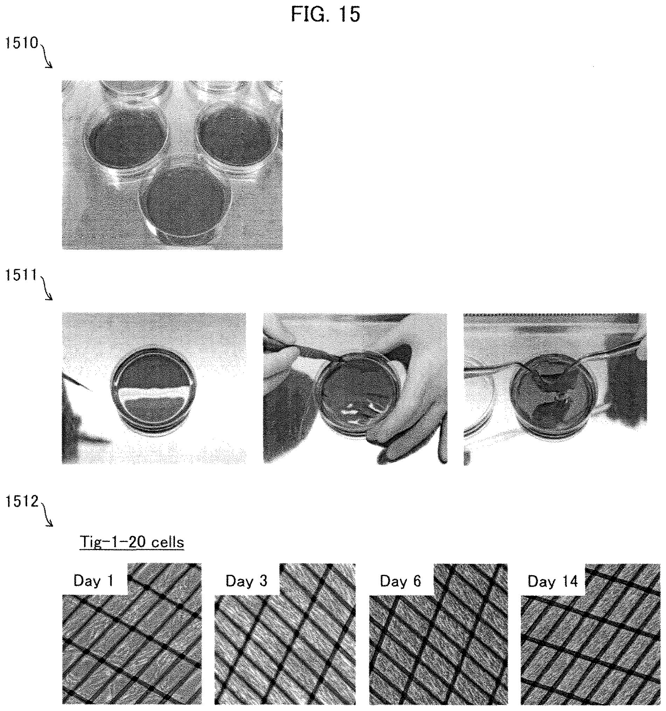

A ring member 1 and a base 3 were prepared by using a 3D printer AGILISTA-3200. Further, the ring member 1 and the base 3 thus prepared were coated with parylene (DPXC, CAS No. 28804-46-8). For a mesh sheet 2, a polyester micromesh sheet (AG00Z3N9) (Amaike Textile) was used. The mesh sheet 2 was sandwiched between the ring member 1 and the base 3, and bonded with use of a PDMS solution. After the support unit 10B that had been completed was subjected to treatment with ethanol and to UV-treatment, the support unit 10B was placed in a 10 cm dish 22. In the support unit 10B which was used in this experiment, a distance from a bottom surface of the dish 22 to the mesh sheet 2 was adjusted to 2.5 mm with use of a 1 mm PDMS plate (1510 of Fig. 15).

Culture conditions (composition of a culture medium) of Tig-1-20 cells are the same as those of Example 1, except that the mesh sheet 2 is not coated.

### <Seeding and microscope observation of Tig-1-20 cells>

First, 3mL of the Tig-1-20 cells (6 × 10⁵ cells/mL) were put on the mesh sheet 2. Thereafter, a covering body 9 was put from above. Then, 5 ml of a cell suspension was added through an injection port 9a of the covering body 9, and a gap between the covering body 9 and the mesh sheet 2 was filled with the cell suspension. Then, 3 days after cell seeding, the covering body 9 was removed after addition of 25 mL of the culture medium to the 10 cm dish 22 in which the support unit 10B was held. The culture medium was changed once every 3 days (35 mL/dish of the culture medium), and culture was finished 14 days after cell seeding.

The cell sheet was cut out from the support unit 10B by using a knife, as shown in 1511 of Fig. 15. After a portion of the cell sheet thus cut out was washed with phosphate buffered saline (PBS) (-), the portion of the cell sheet was treated with 4% PFA for 20 minutes and stained with DiI (37°C, 1 h). After washed with PBS (-), the portion of the cell sheet was fixed, together with a mounting agent VECTASHIELD (VECTOR) which contained DAPI, between a cover glass and a slide glass. The cell sheet thus fixed was analyzed with use of a confocal microscope LSM 800 (ZEISS).

Other portions of the cell sheet which had been prepared by the support unit 10B were each washed with PBS (-), and then treated with 4% PFA for 20 minutes and fixed. Next, those portions of the cell sheet were each treated with 50%, 55%, 60%, 65%, 70%, 75%, 80%, 85%, 90%, 95%, or approximately 100% ethanol for 5 minutes and then air-dried. Then, the portions of the cell sheet were each observed with use of a low vacuum scanning electron microscope Miniscope TM3030Plus (Hitachi).

With regard to cells which constituted the cell sheet that had been cultured in the support unit 10B, viability was examined. Approximately a half of the cell sheet, which had been cut from the support unit 10B, was washed with PBS (-), treated with 0.05% trypsin-EDTA for 7 minutes (37°C), and after removal of trypsin, suspended in 10 mL of the culture medium. The viability of the cells was measured by using Countess II Automated Cell Counter (Thermo Fisher Scientific).

### <Results>

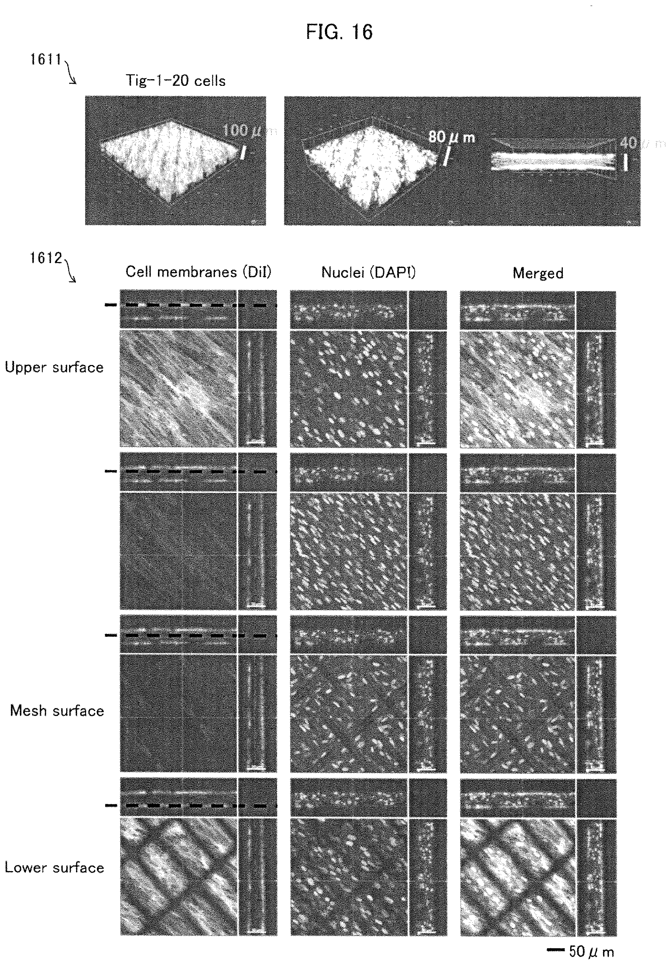

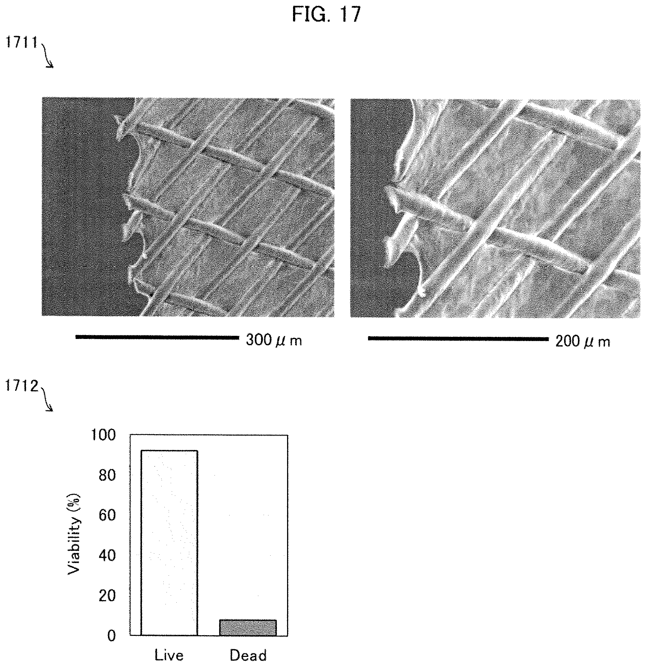

Results of the above observation are shown in 1512 of Fig. 15, 1611 and 1612 of Fig. 16, and 1711 and 1712 of Fig. 17. The Tig-1-20 cells were cultured for 14 days with use of the support unit 10B (1512 in Fig. 15). As a result of analyzing a cross-section of the cell sheet with use of the confocal microscope, the cell sheet was found to have a thickness of approximately 40 µm to 45 µm (1611 and 1612 of Fig. 16). The Tig-1-20 cells were oriented in accordance with the shape of openings of the mesh sheet 2. That is, the Tig-1-20 cells were oriented substantially in parallel to long sides of the openings that were rectangular. Also, cells which were not in a plane of the mesh sheet 2 were oriented in the same direction.

In order to observe the cell sheet with use of the electron microscope, the cell sheet was treated with ethanol. Although this treatment might have caused the cell sheet to shrink and become thinner, it was confirmed that the cells filled the openings of the mesh sheet 2 without a gap (1711 of Fig. 17). With regard to the cells which constituted a three-dimensional cell sheet that had been prepared in the support unit 10B, the viability of the cells was examined with use of a trypsin-EDAT solution and a trypan blue solution. As a result, it was found that approximately 92% of the cells were alive (1712 of Fig. 17). In other words, even in three-dimensional culture, many cells were viable.

### [Example 6: HepG2 cell culture with use of cell sheet production device according to Configuration Example 4]

### <Method>

### <Preparation of support unit 50>

A column body 51 of a support unit 50 was prepared by using a 3D printer AGILISTA-3200. Further, the column body 51 thus prepared was coated with parylene (DPXC, CAS No. 28804-46-8). For a mesh sheet 2, a polyester micromesh sheet (AG00Z3N9) (Amaike Textile) was used. The mesh sheet 2 was bonded to the column body 51 with use of a PDMS solution. After the support unit 50 that had been completed was treated with ethanol and air-dried, the support unit 50 was held in wells 25a of a well plate 25 (12-well plate) and subjected to UV-treatment.

### <Cell culture conditions>

HepG2 cells (Product Number RCB1886) were obtained from RIKEN BioResource Research Center (RIKEN BRC). The HepG2 cells were cultured with use of Dulbecco's Modified Eagle's Medium (DMEM) (GIBCO) which contained 10% fetal bovine serum (FBS) (GIBCO) and 100 units/mL of penicillin and 100 µg/mL of streptomycin (GIBCO). The HepG2 cells were cultured in an incubator under conditions of 37°C and 5% carbon dioxide (CO₂).

### <Cell seeding and cell observation>

On the mesh sheet 2 (circle of ϕ4 mm) of the support unit 50, 1×10⁵ (2 ×x 10⁶ cells/mL × 50 µL/mesh of) HepG2 cells were seeded. Then, after 6 hours from cell seeding, 2 mL of a culture medium was added to the wells 25a. The culture medium was changed once every two days (2 mL/well). As a control, 1 × 10⁵ HepG2 cells were seeded in wells 25a of a well plate 25 (12-well plate).

### <Gene expression analysis>

On tenth day from the start of culture, RNA was extracted from HepG2 cells. The RNA was extracted with use of TRIzol RNA Isolation Reagents (Thermo Fisher Scientific), and cDNA was synthesized by using the RNA thus extracted and a ReverTra Ace qPCR RT Kit (Toyobo). Gene expression analysis was carried out with use of a cDNA sample thus obtained, a DNA primer, a PowerUp SYBR Green Master Mix (APPLIED BIOSYSTEMS), and a QuantStudio 5 Real-Time PCR System (APPLIED BIOSYSTEMS). With regard to genes of albumin and the drug-metabolizing enzyme CYP1A2 which are each known as a maturation marker for human liver, respective expression levels of the genes were analyzed. The respective expression levels of those individual genes were normalized with use of expression levels of 18SrRNA which is known as a housekeeping gene. Value = mean ± SE (N=3).

### <Results>

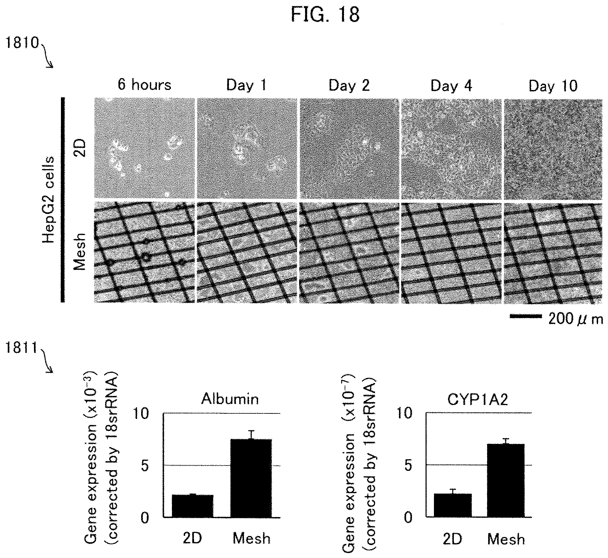

Fig. 18 includes 1810 which shows microscope images of the HepG2 cells. The HepG2 cells adhered to the mesh sheet 2 within 6 hours. Fig. 18 includes 1811 which shows results of the gene expression analysis. The respective expression levels of albumin and CYP1A2 genes were higher when the HepG2 cells were cultured on the mesh sheet 2 (Mesh) of the support unit 50, as compared to a case where the HepG2 cells were cultured on a conventional well (2D).

### [Example 7: Culture of human iPS cell-derived cardiomyocytes with use of cell sheet production device according to Configuration Example 4]

### <Method>

### <Cell culture conditions>

Human iPS cell-derived cardiomyocytes (MiraCell Cardiomyocytes from ChiPSC 12, Takara) were cultured at 37° C in the presence of 5% CO₂, with use of a MiraCell CM Culture Medium according to its manual. Those cells were cultured on a dish which had been coated with human fibronectin.

### <Cell seeding and cell observation>

Prior to cell seeding, a mesh sheet 2 of a support unit 50 was coated with human fibronectin. A fibronectin solution (final concentration: 0.05 mg/mL) diluted 20-fold with PBS (+) was prepared. Then, 50 µL of a resultant diluted fibronectin solution was put on the mesh sheet 2, and left to stand still for not less than 1 hour at 37°C.

Thereafter, the fibronectin solution was removed, and 2 × 10⁴ (4 × 10⁵ cells/mL × 50 µL/mesh of) cardiomyocytes were seeded on the mesh sheet 2 (circle of ϕ4 mm) of the support unit 50. Then, 6 hours later, 2 mL of the culture medium was provided in a well 25a and the cardiomyocytes were cultured. The culture medium was changed once every two days (2 mL/day).

### <Results>

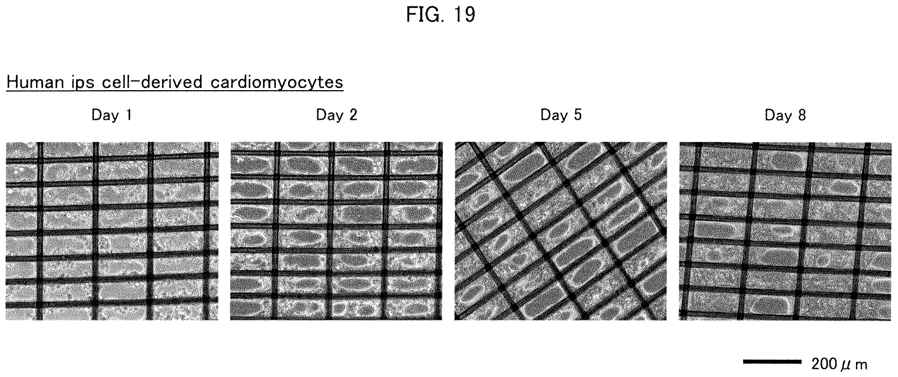

Fig. 19 shows results of the above observation. It was confirmed that the cardiomyocytes had adhered to and grown on the mesh sheet 2 of the support unit 50. The cardiomyocytes retained ability of beating even after having been cultured for 8 days.

### Industrial Applicability

The present invention is applicable to preparation of a cell sheet which is used in regenerative medicine, grafting, and the like.

### Reference Signs List

1 ring member
2 mesh sheet
3 base (holding member)
3b cylindrical placement portion
4, 4a, 4b cell
9 covering body
9a injection port
10, 10A, 10B, 30, 40, 50, 60 support unit
20, 23 container
21, 24, 25a well (container)
22 dish (container)
25, 26 well plate (container)
31, 32 frame body
33 clip (clip member)
41, 51 column body (holding member)
65 pushing pin
66 through hole
67 locking portion
100, 100A, 100B, 100C, 100D, 100E cell sheet production device
200 cell sheet

## Claims

1. A cell sheet production device comprising:
a container configured to contain a culture medium for culturing cells; and
a support unit configured to be removably held in the container, the support unit including:
a mesh sheet that is a substrate to which the cells are caused to adhere and on which the cells are cultured; and
a holding member which holds the mesh sheet such that the mesh sheet floats from a bottom surface of the container,
the support unit being held in the container such that the support unit is at a fixed position in vertical and horizontal directions in the culture medium.

2. The cell sheet production device according to claim 1, wherein the holding member detachably holds the mesh sheet.

3. The cell sheet production device according to claim 2, wherein:
the holding member includes a cylindrical placement portion on which the mesh sheet is placed;
the support unit includes a ring member which has a shape surrounding an outer periphery of the cylindrical placement portion and which is detachably provided to the holding member; and
the mesh sheet has a peripheral edge sandwiched between the cylindrical placement portion and the ring member.

4. The cell sheet production device according to claim 3, wherein:
at least one of the holding member and the ring member includes a locking portion which locks the holding member and the ring member so that the holding member and the ring member do not separate from each other;
at least one of the holding member and the ring member includes a through hole through which a pushing pin for pushing the other one of the holding member and the ring member is caused to pass; and
pushing the other one of the holding member and the ring member releases locking of the holding member and the ring member, so that the holding member and the ring member are separated from each other.

5. The cell sheet production device according to claim 2, wherein the support unit includes:
a pair of frame bodies which are detachably provided to the holding member such that a peripheral edge of the mesh sheet is sandwiched between the pair of frame bodies; and
a clip member which unites the mesh sheet and the pair of frame bodies by clamping the pair of frame bodies.

6. The cell sheet production device according to any one of claims 1 to 5, wherein the holding member has a larger specific gravity than the culture medium.

7. The cell sheet production device according to any one of claims 1 to 6, wherein:
the support unit includes a covering body which covers one surface of the mesh sheet, the covering body being provided with an injection port for injecting a suspension between the mesh sheet and the covering body, the suspension containing the cells; and
a distance between the mesh sheet and the covering body is set so that while coming in contact with both of the mesh sheet and the covering body, the suspension is injected between the mesh sheet and the covering body.

8. The cell sheet production device according to any one of claims 1 to 7, wherein the mesh sheet has openings having a shape elongated in one direction.

9. A cell sheet comprising:
at least two cell layers; and
a mesh sheet between the two cell layers, the mesh sheet being a substrate to which cells are caused to adhere and on which the cells are cultured.
